# EUROPEAN PATENT SPECIFICATION

(11) **EP 1 678 496 B1**
(45) Date of publication and mention of the grant of the patent: **11.06.2014**
(21) Application number: 04768852.8
(22) Date of filing: 12.10.2004
(51) Int. Cl.: G01N 33/487

(54) **METER AND TEST SENSOR BANK INCORPORATING RE-WRITABLE MEMORY**
MESSINSTRUMENT UND TESTSENSORBANK MIT EINGEBAUTEM WIEDERBESCHREIBBAREN SPEICHER
BANC D'ESSAIS ET DE MESURES À MEMOIRE REENREGISTRABLE INTEGRÉE

(30) Priority: 15.10.2003 GB 0324161; 21.10.2003 GB 0324546
(43) Date of publication of application: 12.07.2006
(60) Divisional application: 10180449.0
(73) Proprietor: Inverness Medical Limited, Inverness IV2 3ED (GB)
(72) Inventor: GRIFFITH, Alun, Inverness Scotland IV2 3XQ (GB); COULSON, Alan, Nairn Scotland IV12 4SA (GB); TAYLOR, David, Inverness Scotland IV2 3HW (GB); HAYTER, Paul, Graham, Mountain View CA 94040 (US)
(74) Representative: Tunstall, Christopher Stephen
(86) International application number: PCT/GB2004/004321
(87) International publication number: WO 2005/040793

(56) References cited:
- EP-A- 0 994 350
- WO-A-97/29847
- WO-A-02/078533
- WO-A-03/082092
- WO-A-03/083469
- US-B1- 6 544 475

## Description

This application is related to the following co-pending patent applications: International patent application number PCT/GB02/01599 (Publication number WO02/078533); UK patent application number GB0207609 'Test Strip Vial' filed on 2 April 2002, and UK patent application number GB0207610 'Integrated Sample Testing Meter'.

### BACKGROUND OF THE INVENTION

The present invention relates to meters, systems and devices and methods of using such meter, systems and devices for measuring a quantity in relation to an analyte in a physiological fluid for example, the concentrate of an analyte such as glucose, fructosamine, haematocrit in blood, or the concentration of glucose, fructosamine or other analytes in ISF, glucose or other analytes in urine. The invention also relates to methods of using such meters, systems and devices.

### DESCRIPTION OF THE RELATED ART

Glucose monitoring is a fact of everyday life for diabetic individuals. The accuracy of such monitoring can literally mean the difference between life and death. Generally a diabetic patient measures blood glucose levels several times a day to monitor and control blood sugar levels. Alternative methods of testing which look at other analytes or other fluids such as fructosamine ISF lie within the scope of this invention. Failure to test blood glucose levels accurately and on a regular basis can result in serious diabetes related complications including cardio vascular disease, kidney disease, common nerve damage and blindness.

Many of the glucose meter designs currently available make use of a disposable test strip which in combination with the meter, electrochemically or photometerically measures the amount of glucose in the blood samples. To use these meters the user first punctures a finger or other body part using a lancet to produce a small sample of blood or interstitial fluid. The sample is then transferred to a disposable test strip. The inconvenience of taking several measurements a day, as well as the pain inflicted by currently available lancets, often discourage disciplined and frequent testing. While the fingertip is generally used for sampling blood, due to the rich capillary bed of the skin of the fingertip, the fingertip is also particularly sensitive to pain, due to a rich supply of pain receptors in the finger tip as well. When a puncture is too deep, too close to a recent puncture or not deep enough and requires an additional puncture, the pain increases significantly. Pain may also be increased if the lancet penetrates slowly or is withdrawn slowly. Furthermore, the user may be forced to make a larger puncture than is necessary to form a sufficient amount of blood, due to losses during the transfer between the puncture site and the test strip. The process of measuring blood glucose levels requires several steps and several different accessories including a lancing device, a lancet, a supply of test strips and a glucose meter. Each accessory has a different function. The user typically requires a flat surface available on which to unpack and lay down the accessories within easy reach. This, by itself, poses a challenge for those who need to take measurements while participating in normal every day activities and especially in outdoor activities. Flat surfaces are often not available and this can discourage a person from taking a measurement. This can be disadvantageous because glucose levels may change significantly particularly during an outdoor exercise or activity.

Even if the user can find a flat surface, the user had to carry out the following steps. The user: charges the lancing device with a fresh lancet; opens a vial of strips; removes a strip and inserts the strip into the meter; closes the vial; checks the corrects calibration code on the meter if necessary for example, when a new vial is used a user reads a calibration code from the vial and enters the new calibration code associated with the new strips in that vial, into the meter; picks up the lancing device; lances the skin of the finger or other body part; lays down the lancing device; squeezes or massages the finger to yield an adequate blood sample; transfers the sample to the test strip for analysis; waits for the meter to analyse the sample; removes the strip from the test meter; discards the test strip; and finally repacks all of the accessories. The steps above come as a standard procedure for taking a glucose measurement. Thus, a measurement requires the use of multiple, separate components and the execution of a number of steps requiring manual-user interventions.

Generally, the user is required to place a small volume of sample to a sample receiving area on a test strip. Generally, these test strips are quite small and the sample receiving area is therefore even smaller. This transfer step is a difficult task for many users. Moreover, there has recently been a trend towards the use of test strips requiring even smaller amounts of sample which allows the use of smaller punctures and therefore less painful lancing. However, the use of smaller samples increases the difficulty in transferring the sample to the sample receiving area on the test strip. This is especially difficult for users with poor eyesight, a common complication for diabetes. There is therefore a need for a test strip which aids the introduction of a blood sample to the appropriate point on the test strip.

The pain, inconvenience, cost, slowness, complexity and lack of discreteness of taking a blood glucose measurement is problematical to the frequent monitoring of blood glucose levels. Patients often do not comply with instruction documents recommendations to frequently test glucose levels due to the numerous obstacles involved. It is an aim of the present invention to provide, at least in part, a solution to alleviate the above problems.

Errors can occur if a user fails to or incorrectly enters a new calibration code when a new vial of strips is used. In the manufacture of test sensors, and in particular in the manufacture of electrochemical test sensors, variations can occur in the sensors. This can mean that whilst the relationship between blood glucose, for example, and test result, typically in the form of a current measured, is predictable, it is not always predictable in the same manner. The relationship between blood glucose and current measured can be approximated to a linear relationship involving a slope (c) and intercept (m). From batch to batch, and to a lesser extent across batches, the slope and intercept defining the relationship between blood glucose and current measured can vary. To ensure that the correct relationship is used to determine the blood glucose from the current measured during a test, a calibration code defining the value of the slope and intercept is entered into the meter each time strips from a new vial, and hence a different batch, are used.

It is therefore important for the correct calibration code to be entered to ensure that any batch-to-batch variations are taken into account in the conversion from a raw test result to final test result. Thus, further difficulties are encountered by a user when opening a vial of test strips for the first time. In this case, the user must read the calibration code for that vial of strips from a man readable label on the side of the vial and, remembering this calibration code pick up the meter and switch it on, moving to the correct menu on the meter screen to input the new calibration code for the newly opened vial of strips. This data input step can present some difficulty for young diabetics who may have difficulty remembering to carry out this step, or indeed difficulty in carrying it out because they fail to read the numbers on the side of the vial correctly or remember these numbers incorrectly or indeed type these numbers incorrectly into the meter. Similarly, for visually impaired or elderly diabetics this data transfer step can also be problematical for the same reasons. It is an aim of the present invention to alleviate or provide, at least in part, a solution to the above problems.

In the manufacture of test sensors such as strips, variations can occur during manufacture, which can result in variations in the slope and intercept of the relationship-linking test results measured on the strip and blood glucose. A calibration code is derived after manufacture for each batch of test sensors. Before distribution of strips, this code is linked in some way with a set of strips from that batch so that the code can be used during testing using those strips. Unfortunately this link may simply involve placing a calibration code on a card in the same packaging as the strips. Test sensors such as strips are typically loose within a vial or cartridge, so there is a possibility of a mix-up occurring during manufacture, packaging, distribution or use, and the wrong calibration code being used in the meter in connection with a particular set of strips for tests. Other solutions than manual entry of calibration code include placing a calibration code on each test strip or fixedly locating a calibration code readable by the meter on a collection of test strips such as the vial with a machine-readable bar code or memory chip. For example, a bar code sticker is affixed to the strip container or a calibration code strip is provided with that batch of strips. Nevertheless there still exists the possibility for misreading of the calibration code by the meter since there is no intelligent check of the information being uploaded to the meter. Furthermore, provision of a calibration code on each strip is likely to be prohibitively expensive and discourage users from testing frequently because of the extra expense incurred on each strip. Where a separate memory device, such as a smart card, is provided for storing test results and calibration codes, the problem remains of possible mix ups between strips so that the wrong strips are associated with that memory device and hence calibration code. It is an aim of the present invention to alleviate or provide a solution, at least in part, to some of these problems.

One example of this can be seen in international patent application PCT/US97/02166 (Publication number WO97/29847). A vial of strips bearing a machine-readable calibration code is inserted into a meter. In addition, this patent application also describes the calibration code, such as a bar code or memory chip having calibration code being in an optionally erasable format so that once read and used for a predefined number of strips, the total number of strips in the vial initially, for example, the machine calibration code is erased. This prevents further use until a new vial bearing a calibration code is inserted.

Roche Diagnostics Limited, Lewes, East Sussex, UK produce a meter ACCU-CHEK® Compact having a seventeen-strip drum insertable in a meter. The drum carries a machine readable bar code, which can be read by the meter on insertion of the drum into the meter. As the user activates the meter ready for testing, a strip is delivered from the drum to a test port ready for testing. A user conducts a test by application of a blood sample, and once testing is finished, the strip is removed from the meter by a user for disposal.

Although this drum and the vial described in WO97/29847 both carry a calibration code readable by the meter and therefore address the issue of a user having to enter a calibration code into the meter, such systems nevertheless have limitations. For example, if for whatever reason, the user removes a strip from the meter before all the strips are dispensed, the meter may not recognise on re-insertion of a part-used vial or drum that it has already been part-used and therefore not all the strips it expected to be present in the vial or drum are there. Also the part used vial or drum may have been open to the atmosphere for longer than recommended. There is therefore a need to try and ensure that the strips contained within the vial or drum are not over exposed to moisture, and at least a user is warned in this eventuality.

Other limitations exist in meters. For example, once a number of meters have been placed in the field with users, it is extremely difficult, time consuming and expensive to replace those meters in order to upgrade software, for example, to improve the software or to correct errors. Furthermore, whilst meters are able to store a number of test results, typically the amount of memory allocated for such a purpose is limited. Collating test results over a long period of time can be very useful in assessing the success or not of a patient's treatment. Other information might be useful in connection with test results such as food intake (type of food intake, amount time and date), exercise (type, amounts, time and date), medication (type, amounts, time and date), general state of health (nature of condition, time and date). The amount of memory available in meters may be too limited to accommodate results over extended periods of time. The ability to store and extract such data for later analysis is limited in current meters.

During a lifetime each diabetic accumulates a vast number of test results. A more intimate understanding of the diabetic's condition and how it can best be controlled can be gleaned by analysis of an accumulated set of test results, over a long period of time, for example, over periods of three, six, twelve months etc. Whilst meters often store the last two hundred and fifty or five hundred results, the oldest result is lost when a new one is added because of limited memory size. Furthermore, there is currently no convenient way of downloading such results to a user or clinician's computer for further in-depth analysis. Therefore, the opportunity to review and investigate trends in a diabetic's results over long periods of time is currently being lost. It is an aim of the present invention to alleviate or provide a solution, at least in part, to some of the above problems.

US5366609B 'White, et al' filed 8 June 1993 (Applicant BOEHRINGER MANNHEIM Corporation) describes a bio-sensing meter able to receive a sample strip and in addition having a separate plug-able memory key insertable into the meter including a plurality of stored parameter values and procedure routines that control the algorithm used in the meter. Replacement of the plug-able memory key with a memory key containing alternative procedures and parameters enables the bio-sensing meter to carry out substantially modified test procedures without a requirement for modification of the structure of the meter.

US6413213B 'Essenpreis, et al' filed 18 April 2000 (Applicant ROCHE DIAGNOSTICS Corporation) describes a system including a meter with a Read Only Memory ROM circuit, a test strip and a registry. The meter has an identifier corresponding to a subscription user. The ROM circuit is provided with test strips and contains calibration data and an identifier. The registry checks for valid association of the meter identifying a ROM circuit identifier and a user identifier. If a valid association is established, the registry activates the meter for a subscription period. The meter thereafter validates the ROM circuit so that the ROM circuit may only be used with that meter, thereby discouraging fraudulent conveyance of ROM circuit and test strips.

US6454708B1 'Ferguson, et al' filed 9 January 2000 (Applicant NEXAN Limited) describes a portable remote patient tele-monitoring system using a memory card or smart card. The system uses a cordless disposable sensor to measure ECG respiration, skin temperature and motion and a connector which connects a memory card or smart card for storage of the measured data. After a period of time the memory card or smart card is removed and inserted into a monitoring device which reads the stored health parameter of the subject. Monitoring device includes a base station including a memory/smart card reader and is connected to conventional telephone lines for transferring the collected data to a remote monitoring station.

International Patent Application WO99/51989 'Catt, et al' filed 26 March 1999 (Applicant UNILEVER Plc) describes a method for determining the time of maximum fertility in the mammalian ovulation cycle. An electronic monitor used to log data in connection with this method includes interface means to communicate with electronic data transmission means such a smart card or floppy disk. The data transmission means is used to transfer information to a computer such as a PC in the home or in a clinic. The smart card or floppy disk can also be used to change or supplement the algorithm or data store in the monitor.

US Patent Application Number 2003/0032077 to 'Takehito, et al' filed 6 August 2002 (Applicant NIPRO Corporation) describes a recording medium having a sensor circuit unit generating a current in accordance with the blood glucose level in blood. Data is written to an EEPROM as a memory unit. A control unit transmits the blood glucose data to a portable terminal (mobile telephone or cell telephone) on which the recording medium is mounted. The recording medium receives power from the portable terminal and can be utilised when mounted on the portable terminal as a blood glucose monitoring apparatus and for providing bi-directional service between the portable terminal and the server managing blood glucose information received from the portable terminal via a network.

National Diagnostic Products (Australia) Pty. Limited, Killara, NSW 2071, Australia describe a blood glucose meter BETACHEK G5 as a meter with memory card technology. A memory card is included with each pack of tests allowing access to two hundred and fifty memories in the G5 meter by simply inserting a full memory card to review stored results. The memory card can also be used to download data to a PC for analysis. Fifty results are permanently stored on each memory card with two hundred and fifty memories being accessible in the G5 meter.

WO 03/083469 provides an integrated sample testing meter having a test strip cartridge and a memory chip fixed to the cartridge.

### SUMMARY OF THE INVENTION

### BRIEF DESCRIPTION OF THE DRAWINGS

The novel features of the invention are set forth with peculiarity in the appended claims. A better understanding of the features and advantages of the present invention will be obtained by reference to the following detailed description and accompanying drawings that sets forth illustrative embodiments, in which the principles of the invention are utilised. The drawings and detailed description are shown by way of example only.
Figure 1A shows a schematic, block diagram of an example system incorporating a meter and strip bank with READ/WRITE memory device.
Figure 1B shows a schematic, block diagram of a system incorporating a meter, strip bank and separate READ/WRITE memory device in accordance with the present invention.
Figure 1C is a schematic, block diagram illustrating another example system incorporating a meter, a strip bank and first READ/WRITE memory device and separate second READ/WRITE memory device.
Figure 2 shows a perspective, schematic view of an example of a meter and strip bank of Figure 1A.
Figure 3 shows a perspective, schematic view of a further example embodiment of a meter and strip bank.
Figure 4 shows a perspective, schematic view of a further example embodiment of a meter and strip bank.
Figures 5 and 6 respectively show plan and side elevation views of an example embodiment of a strip bank in the form of a substantially circular disc incorporating several strips and a READ/WRITE memory device that can be used in a system in accordance with the present invention.
Figure 7 shows in perspective view, example embodiment of a meter, a strip bank and a separate card incorporating a READ/WRITE memory device in accordance with the present invention.
Figure 8 illustrates in plan view the base of the meter of Figure 7 with slots 54 and 56 sized and shaped to receive strip bank 10 and card carrier 30.
Figures 9A, 9B and 9C illustrate in perspective view a meter, a strip bank incorporating a first READ/WRITE memory device and a card incorporating a second READ/WRITE memory device.
Figure 10A illustrates a plan view of an example embodiment of a meter incorporating a lancet and a strip bank that can be used in a system in accordance with the present invention.
Figure 10B shows a partial cut away view of the meter of Figure 10.
Figure 11A shows a perspective view of another example embodiment of a meter and strip dispenser incorporating a smart chip 18.
Figure 12 illustrates a plan view of a strip bank incorporating a READ/WRITE memory device for use in the meter of Figures 10 and 11.
Figure 13 shows in plan view of an example READ/WRITE memory device illustrating contact pin connections that can be used in a system in accordance with the present invention.
Figure 14 shows a schematic diagram providing further details of the contacts of the READ/WRITE memory device of Figure 13.
Figure 15 illustrates in perspective view a strip cartridge for carrying a stack of strips and a smart chip (READ/WRITE memory device) that can be used in a system in accordance with the present invention.
Figure 16A, 16B and 16C illustrate flow diagrams showing the steps, which may occur, on insertion or connection of the strip bank to the meter in accordance with the present invention.
Figure 17 shows a flow chart illustrating the possible steps to be taken following a test and immediately prior to final power off of the meter.
Figure 18 illustrates perspective views of a stack of strips and cassette that can be used in a system in accordance with the invention and details the information which may be shown on the cassette.
Figure 19 is table showing the types of information which may be loaded from the READ/WRITE memory device to the meter and from the meter to the READ/WRITE memory device that can be used in a system in accordance with example embodiments of the present invention.
Figures 20A and 20B illustrate front elevation and side elevation views of a meter 20 and Figure 20C illustrates a plan view and close up of a smart card 40 that can be used in a system in accordance with the present invention.
Figure 20D illustrates further examples of smart card 40 in plan view in accordance with Figure 20A to 20C.

### DETAILED DESCRIPTION OF EXAMPLE EMBODIMENTS OF THE INVENTION

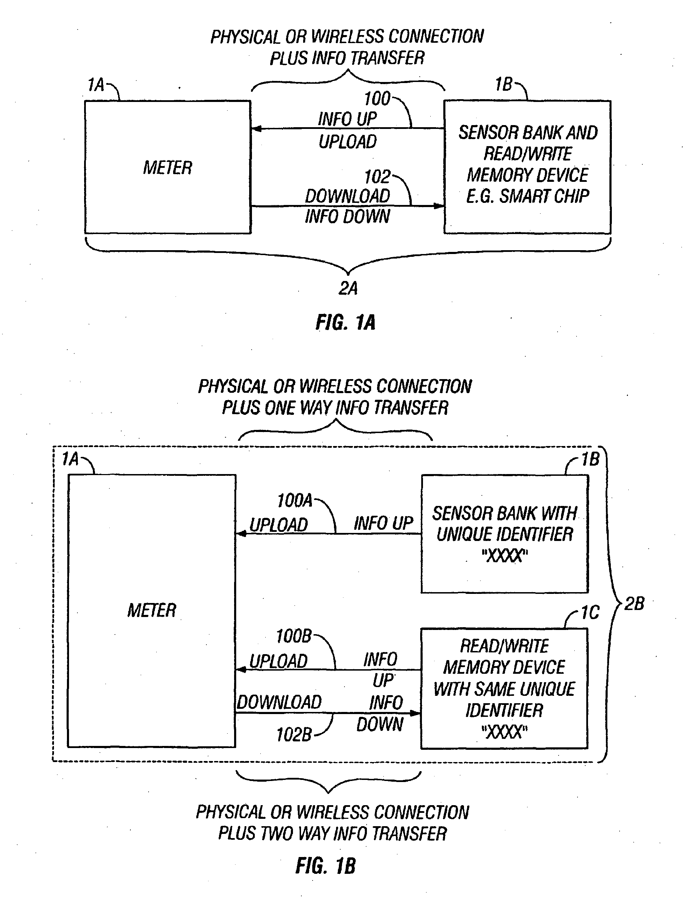

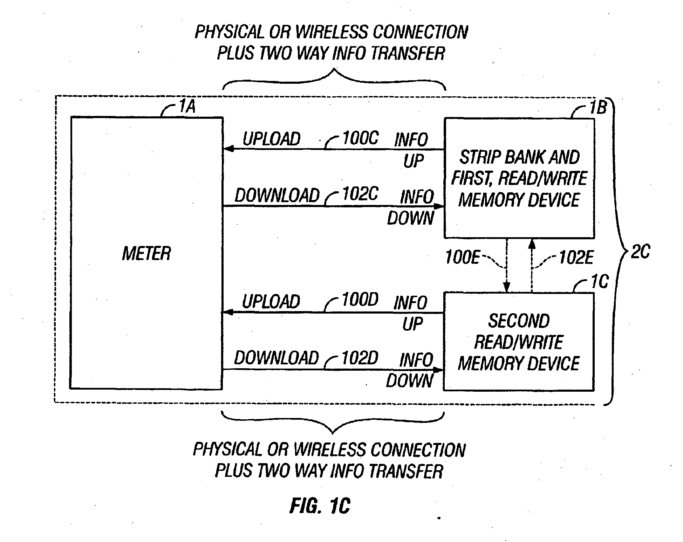

Figure 1 shows a schematic, block view of a system 2A suitable for testing for an analyte in a sample fluid. This system 2A comprises a meter 1A and a sensor bank incorporating a READ/WRITE memory device 1B. In this case sensor bank 1B comprises, at least initially before use, more than one sensor for use in testing for an analyte. The sensor bank and READ/WRITE memory device are fixedly attached together during manufacture so that the information contained in the READ/WRITE memory device, be that information to be uploaded (see arrow 100) to meter 1A or information to be down loaded from meter 1A (see arrow 102), is fixedly, typically permanently, associated with the sensors contained within the sensor bank. In one embodiment, removal of the READ/WRITE memory device can render the sensor bank unusable because the physical or wireless connection between sensor bank and READ/WRITE memory device is broken or unconnectable.

It is to be understood from the disclosure herein that where the term test 'strip' is used, a test sensor of differing shape than a strip can be envisaged. Thus, a test strip is one example of a test sensor as described in this disclosure.

It is also to be understood that the terms re-writable memory and READ/WRITE memory are used interchangeably herein to mean memory which can be written to and read from. Typically, the re-writable memory device will include re-writable memory such as EEPROM or it may consist exclusively of re-writable memory such as EEPROM. Thus, the re-writable memory device may include re-writable memory such as EEPROM and other components such as a microprocessor or microcontroller as will be described hereafter. Such a device may be known as a 'clever' memory. Other components which may be included in the re-writable memory device include components to facilitate wireless read/write operations. In one case these components may facilitate read/write operations on the approach of the re-writable memory device to the meter as will be described hereinafter. Such a re-writable memory device may be known as a 'proximity' memory device. These types of memory may both be found in one device.

Examples of a smart chips which might be used in the invention include the AT24C01ASC, AT24C02SC, AT24C04SC, AT24C08SC and AT24C16SC which are respectively 1K Bits (128x8), 2K Bits (256x8), 4K Bits (512x8), 8K Bits (1024x8) and 16K Bits (2048x8) bytes two wire serial EEPROM smart card modules available from Atmel Corporation of San Jose, California USA, or CAT24C02C, a 2K Bit Serial CMOS EEPROM from Catalyst Semiconductor Inc., Sunnyvale, California USA; IS24C02-3 2K (256x8) Serial Memory, IS23SC4442 2K (256x8) Secure Serial EEPROM, IS23SC4408 8K (8 bit MCU available with 8K EEPROM), MCU based smartcard IC from Integrated Silicon Solutions Inc. Santa Clara, California, USA. Other sizes of re-writable memory lie within the scope of this invention.

Sensor bank and READ/WRITE memory device 1B incorporate a plurality of sensors and a READ/WRITE memory device uniquely or permanently associated or attached to the sensor bank during manufacture. The READ/WRITE memory device contains re-writable memory such as a smart chip for example an EEPROM. Information can be read from and written to the READ/WRITE memory device by the meter 1A when the sensor bank 1B communicates with the meter either via a physical or wireless connection.

The connection between meter 1A and combined sensor bank and re-writable memory device 1B may be a real physical connection or a wireless connection. Information may be uploaded from sensor bank 1B to meter 1A from the re-writable memory device via a physical or a wireless connection. Similarly, information can be downloaded from the meter to the re-writable memory device in the sensor bank via a real physical connection or via a wireless connection. (see information links 100,102)

In one example embodiment, the re-writable memory device is physically, fixedly connected to the sensor bank to form an integral sensor bank and re-writable memory device 1B. Thus the sensors in that sensor bank are physically associated with that re-writable memory device and any information contained within it. Thus a calibration code written into the re-writable memory is uniquely associated with the test sensors in that bank and can be used in converting a raw test result to a final test result for these sensors. Furthermore, since the memory is re-writable, information can be downloaded to the memory device, such as raw test result, final test result, time, date and so on. Examples of types of sensor bank and re-writable memory device will be described in more detail later.

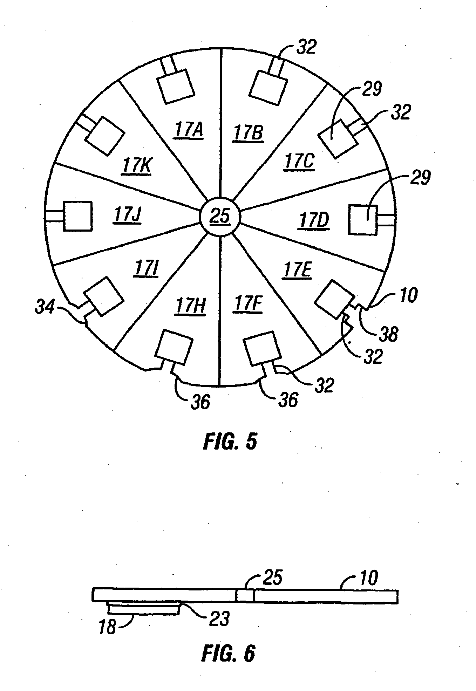

The sensor bank may contain several sensors singulated, i.e. separated sensors, and stacked together or non-singulated sensors, for example separate and distinct sensors formed on a single base or indeed several separate sensors connected to each other in series (one example of this is seen in Figure 5). The sensor bank is in effect a reservoir of unused test sensors and it may be in the form of a container, a vial, cartridge, dispenser (including sensor dispensing means), card and so on as might be envisaged by those skilled in the art from the information contained herein.

For example, as will be well understood by one skilled in the art, the sensor bank may contain a number of separate sensors such as strips located within a housing such as in a vial of strips or a cartridge or other receptacle containing a number of separate sensors, often such sensors being stored in the receptacle in a stack. Typically, where the sensor bank comprises a vial of strips a user will remove a single strip from the sensor bank and insert this into the meter. Where the sensor bank comprises a cartridge containing, for example, a stack of strips, a strip delivery system may also be provided either within the sensor bank or within the meter for engaging with the sensor bank for delivering a strip ready for use, for example, to a test port. Sensors are typically provided in the form of strips although other types of sensors can be envisaged from the disclosure contained herein.

Alternative embodiments can be envisaged in which a sensor bank and a separate re-writable memory device are both provided with the same common unique identifier (for example 'XXXX') so that the sensors contained within the sensor bank are uniquely associated with that unique identifier and hence with that particular re-writable memory device even though there may be no physical connection between the two. An example of such an embodiment is shown in Figure 1B. A meter 1A has an information link 100A with a sensor bank 1B, having a unique identifier 'XXXX'. The unique identifier may be hard-wired or otherwise encoded into respective parts.

A system of the invention is illustrated in a system 2B for measuring an analyte comprises a meter 1A, a sensor bank 1B and a separate re-writable memory device 1C, the sensor bank 1B and re-writable memory device 1C being provided with the same common unique identifier. Information can be exchanged between meter 1A and the re-writable memory device 1C via information links 100B and 102B. The sensors within the strip bank may also be provided with the same common unique identifier 'XXXX', thus providing extra security in the system.

In particular information can be exchanged if the meter sees the same identifier code on the test sensor bank and the separate re-writable memory device and, optionally, the sensor. Thus a calibration code, for example, written into the re-writable memory is clearly associated with the sensors in the bank having the same identifier code. This can be uploaded to the meter for use in tests using sensors from that bank. Furthermore information resulting from the test such as revised strip count information and so on can be downloaded to the re-writable memory device on the bank.

The connection between the sensor bank 1B and the meter 1A and the connection between the re-writable memory device 1C and the meter 1A may be real, physical connections or wireless connections. In use, the meter 1A will upload information from the sensor bank 1B and re-writable memory device 1C about the unique identifier. Once the meter recognises that the unique identifier is the same for sensor bank 1B and re-writable memory device 1C, and optionally, is the same for the sensor itself, further interactions between the separate components take place, which allow a measurement to be made. These interactions may include uploading the calibration code from the re-writable memory device 1C for use in a test conducted using a sensor from sensor bank 1B, the memory device 1C and sensor bank 1B having the same common unique identifier.

In effect, the sensor bank 1B with unique identifier 'XXXX' and the re-writable memory device 1C with identifier 'XXXX' forms a combined sensor bank and re-writable memory device, the separate components of which are uniquely or clearly associated with one another such that the sensor bank is unable to be accessed by meter 1A unless meter 1A has previously, substantially simultaneously or soon thereafter, recognised a re-writable memory device with the same unique identifier. This recognition enables information within that re-writable memory to be associated with test sensors in that bank. Furthermore, information from tests using sensors from that bank can be written back to that re-writable memory for collection and optionally for transport to a computer with an appropriate READ/WRITE memory device reader for later analysis.

Figure 1C illustrates an example system 2C. A strip bank 1B has an integral first re-writable memory device. In other words the first re-writable memory device is physically, fixedly attached to the strip bank or forms part of the strip bank. The strip bank with integral first re-writable memory device is physically or wirelessly connected to meter 1A to provide information link 100C for uploading information and information link 102C for downloading information to the strip bank 1B. A second re-writable memory device 1C is also provided in system 2C. The second re-writable memory device is physically or wirelessly connected to meter 1A via upward information link 100D and downward link 102D. Optionally, second re-writable memory device is also able to exchange information with first re-writable memory device via information links 100E and 102E. The strip bank 1B may be physically connectable to or insertable into meter 1A or provided with means for enabling a wireless connection with meter 1A. Similarly, the second re-writable memory device 1C may be physically connectable to or insertable into meter 1A or provided with means for enabling a wireless connection with meter 1A. Similarly, meter 1A is provided with means for connecting to integral strip bank and first re-writable memory device 1B, either physically or wirelessly, and to second re-writable memory device 1C, either physically or wirelessly. Such means may be in the form of an external snap-fit connection mechanism or recess for telescopic-like insertion of devices 1B and 1C into the meter 1A or transmission/receiving means such as an aerial and associated circuitry for facilitating wireless transmission between devices 1B and 1C and meter 1A. The transmission means may be a wire connection when a re-writable memory device is physically connected to the meter. Input and output circuits (not shown) may be provided in the meter for reading information from and writing information to the re-writable memory device.

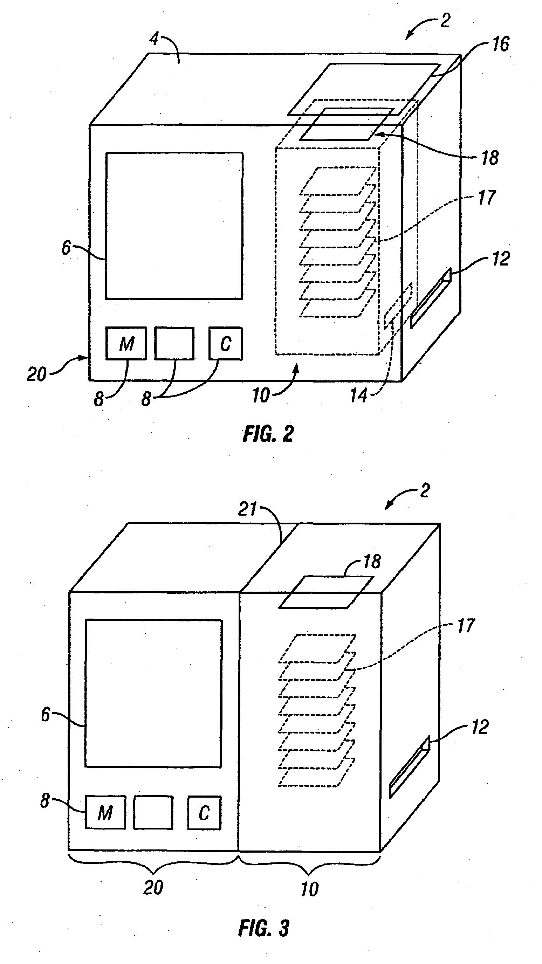

Figure 2 illustrates a more specific example of an embodiment of a meter and sensor bank system of Figure 1A. Here meter 20 comprises a housing 4, a display 6 and control buttons 8 for operating the meter. A sensor bank is provided in the form of a cartridge 10 having a stack of strips 17 contained therein and a smart chip 18 such as an EEPROM affixed thereto. Thus, a re-writable memory device is provided in the form of smart chip 18 fixedly attached to strip cartridge 10. Automatic strip delivery means (not shown) are provided for delivering a strip 17 via an aperture 14 in cartridge 10 to an external aperture 12 in housing 4 ready for testing by a user. Test port 12 typically has associated with it strip connection means, for example, contact pins for contacting with a sensor mechanism on the strip when the strip is delivered to port 12. Alternatively, or in addition a strip could simply be delivered to aperture 12 for insertion in an alternative strip port elsewhere on the meter (not shown).

Sensor bank 10 is insertable into meter 2 via a door 16 in meter housing 4. Internal to meter 2 are connections (not shown) for connecting with smart chip 18 to provide upward and downward information links 100 and 102 as illustrated in Figure 1A.

Manufacture of test sensors 17 is a strictly regulated process to ensure consistency in manufacture. As has been explained in the preceding sections variations can occur in manufacture from batch to batch. A calibration code is used to even out any differences from batch to batch in the algorithm used to calculate blood glucose concentration, for example, from the current measured on a strip. Once a batch of strips has been manufactured, a calibration code will be determined. A smart chip will be programmed with this information and optionally other information as will be described later. In a strictly regulated and controlled process the smart chip 18 will then be tested to verify the contents of its memory and if this is correct the smart chip will be affixed to the cartridge containing a set of strips from the batch which has that calibration code. Thus, the calibration code is physically associated with the strips in the cartridge by virtue of its presence in the smart chip. In the particular embodiment in which aperture 12 functions as a strip port for testing, the cartridge 10 is a closed unit delivered to a user. Therefore there is virtually no possibility of the smart chip and strips being incorrectly associated with one another following manufacture and before or during use.

In the embodiment in which apertures 14 and 12 function as strip delivery apertures (and not strip ports for testing) a strip is delivered from cartridge 10 or withdrawn by a user ready for insertion into a test port elsewhere on the meter, there is a reduced likelihood of a mix-up of strips with an incorrect calibration code. This is because the smart chip will already be within or adjacent to the meter and a user would have to obtain a strip from a totally separate vial or cartridge in order to facilitate a mix-up of strips and calibration code, a relatively unlikely scenario. Thus the former arrangement virtually eliminates the risk of a test strip being incorrectly associated with the calibration code from a particular smart chip, whilst the latter embodiment reduces the risk significantly.

The presence of re-writable memory chip 18 allows information to be downloaded from the meter to the smart chip as well as uploading of information from the smart chip to the meter. Examples of information which can be uploaded from the smart chip to the meter include but are not limited to: 'calibration code' information; 'number of strips' information; 'strip bank opened' information; 'shelf life expiry' information; 'lot/batch/strip identification' information; 'instructions' for the user, 'software (upgrades or corrections)'; 'control solution' information; 'country code or country market flavouring' information; 'personalisation' information, for example, a meter can be flavoured to interact in a different way for young and adult patients or from one patient to the next; 'self learning parameters', for example, information may be used from previous test results to develop an improved algorithm or correction to the algorithm for a particular user for example to take account of the haematocrit response of a particular user or response of a particular user to other factors such as food intake and exercise; 'revised or new analyte calculation algorithm parameters' which may be factory set for a new meter or based on previous test results for an existing user; 'previous test results'; or 'CHECKSUM' to check the memory contents; parameters for the user interface; information about new products; information about performance of present product.

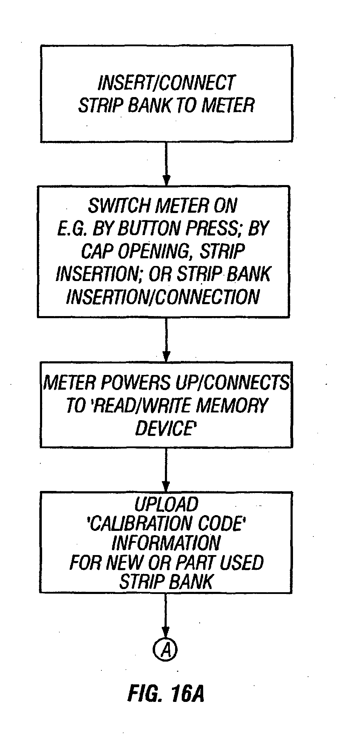

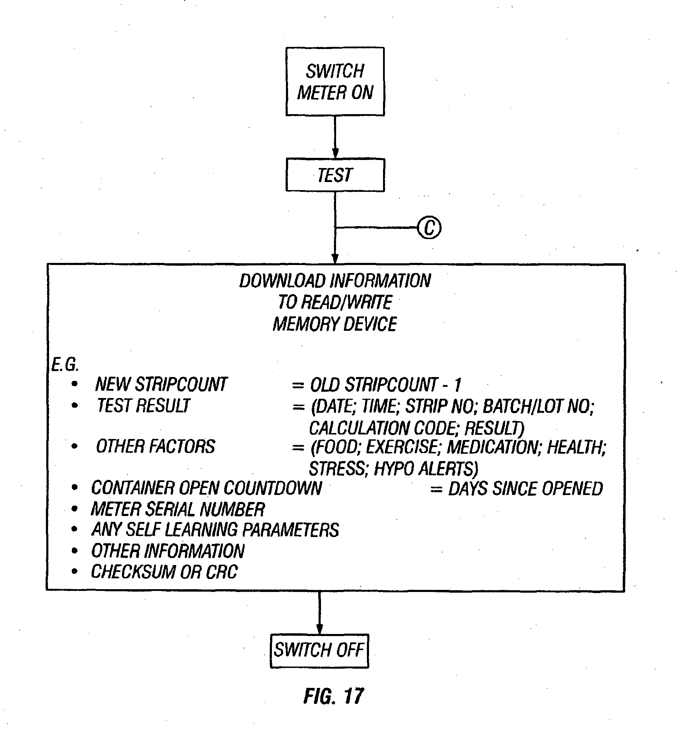

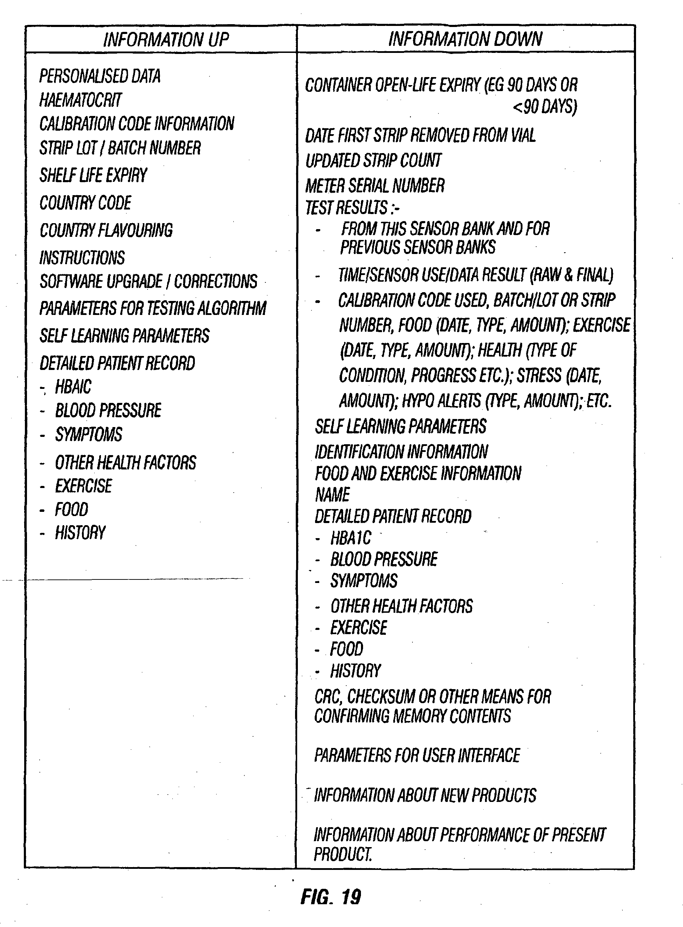

Typically when a meter is switched on, for example by a button press or a cap opening or a strip insertion or a strip bank insertion or connection, the meter reads the re-writable memory device and one of the above types of information is uploaded to the meter from the re-writable memory device. Typically this will include at least the calibration code information although other types of information such as the number of strips initially and remaining unused may be uploaded. In addition or if the user wishes a test to be carried out then he or she can do so before switching the meter off. Typically following a test, information will be downloaded to the re-writable memory device such as 'NEW COUNT = (OLD STRIP COUNT - 1).' Thus, the next time the meter is switched on a new strip count related to the number of unused strips, or the number of useable strips should a mechanism be provided for detecting an unusable strip, can be uploaded to the meter. Other information which could typically be stored in the READ/WRITE memory device include: but are not limited to the following: the result of the test including for example the 'test result, current measured, calibration code used, date, time, strip number, batch/lot number;' other information which has been entered by a user such as information related to food such as 'date, food type amount;' exercise such as 'date, exercise type, amount;' medication such as 'date, medication type, amount;' health such as 'type of condition, progress;' stress such as 'date, type of condition, progress;' hypo alerts such as 'date, type of alert, length of time;' 'container open count down' in other words the number of days since the sensor container was opened; meter serial number; any self learning analyte calculation algorithm parameters that have been calculated by the meter from previous test result; a CHECKSUM for checking the memory contents; parameters for the user interface; information about new products; information about performance of present product. Subsequently the meter can be switched off. Examples of the type of information which can be uploaded and downloaded to the re-writable memory device, and the methodology which might be followed are shown in figures 16A 16B, 16C and figure 17 and figure 19.

Figure 3 shows a sensor bank 10 and a separate meter 20. In this case sensor bank 10 is a cartridge of strips 17 which is provided with means (not shown) for connecting to meter 20 at 21 such as a snap-fit locking arrangement. Here the cartridge 10 and meter 20 are sized and shaped to correspond to one another so as to provide a combined device 2 having a smooth outer profile. The housing of strip bank 10 may be made from polypropylene or polypropylene based plastic or other plastic or other material having good moisture vapour barrier characteristics. A re-writable memory device is adhered to this material using an adhesive which may be a cold cure adhesive, pressure sensitive adhesive, heat seal adhesive or hot melt adhesive. Cold cure adhesives, which cure to a certain bond strength say 40% of total within a fixed period of time, say, 600mS can be used.

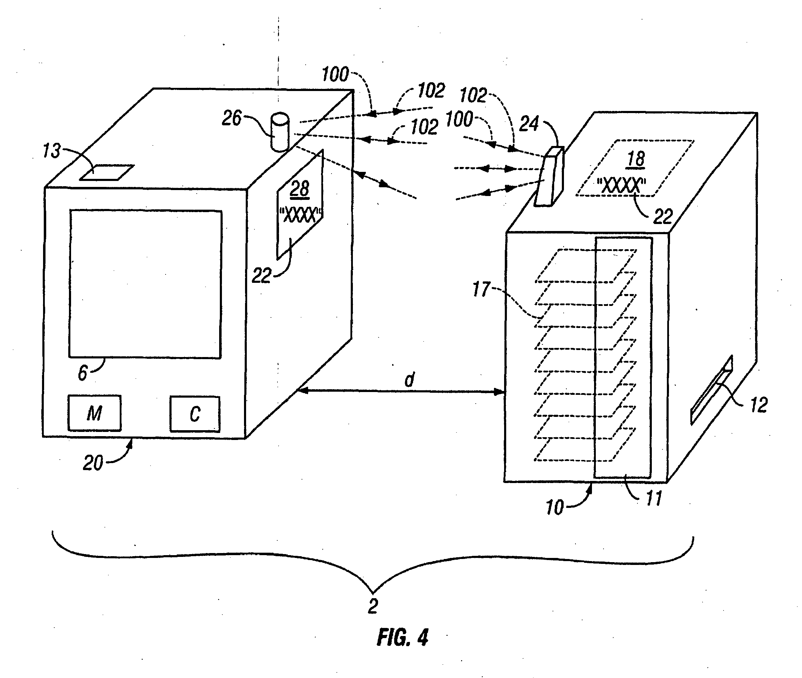

Figure 4 illustrates an example embodiment of a system 2 comprising a meter 20 and a sensor bank 10 which communicate with each other via a wireless connection to provide information links 100, 102. Sensor bank 10 contains a stack of strips 17 which are delivered ready for a testing via aperture 12. Once the strip emerges from aperture 12, it is removed by a user, and inserted into strip port 13 ready for testing. A window 11 is provided in the housing of cartridge 10 so that a visual indication of the number of strips remaining in the strip bank is available to a user. Such a window could be placed in any of the sensor banks of the present invention but finds particular use in sensor banks in which a stack of sensors decreases in size strip by strip providing an observable indication of the reduction in the number of strips. The window may be made from any suitable transparent material, one such material is transparent polypropylene or polypropylene based material, having good moisture vapour barrier characteristic. A correspondingly located window maybe located on the meter for viewing inside the sensor bank when it is located within the meter.

A smart chip 18 typically a 2K Byte EEPROM, is fixedly attached on the side of sensor cartridge 10 and provided with a unique identifier code 'XXXX'. The identifier code may have any number of digits, letters and/or number or be in any base or code so as to provide a unique identifier code recognisable by another device. An aerial and receiver 24 is provided for transmitting information wirelessly via information links 100, 102. Meter 20 is provided with a microprocessor 28 capable of recognising unique identifier code 22 from smart chip 18. Typically smart chip 18 sends the calibration code for strips 17 to meter 20 via information links 100. Similarly, information concerning the 'test results', 'number of strips' and so on can be down loaded to the re-writable memory device 18. The number of strips information is optional in this embodiment because of the availability of a window through which a user can observe the physical reduction in the number of strips. Meter 20 is provided with transmission and receiving means 26 to provide and receive information over information links 100, 102.

In a further embodiment, a system according to the invention comprises a meter and a separate sensor bank having a mechanism for loading strips into the meter when the sensor bank is docked with the meter. This embodiment delivers the calibration code information to the meter transparently without any additional steps.

A sensor bank, for example a vial of strips is provided with a proximity memory and the meter is provided with means for reading/writing to this memory. A specially designed strip vial contains a proximity memory and a means to load strips into the glucose meter for example a strip delivery mechanism, which delivers strips on docking with the meter. Placing the vial close to the meter to load a strip, provides the opportunity for the meter electronics to read or write the contents of the proximity memory.

Typically BiStatix^{™} memory and other proximity memory devices provide radio frequency identification (RFID) as an alternative to bar code identification. RFID works on an inductive principle. A magnetic field is generated at a predetermined frequency. When a proximity memory device enters the field, a small electric current forms in the circuitry of the memory device consisting of a coil and a capacitor. Power is provided to a microprocessor, which modulates the magnetic field to transmit data pre-programmed into the chip to the reader. In the present example the reader would be mounted on the meter and the RFID device would be placed on the vial of strips or strip cartridge.

One example of the proximity memory which might be used, is the BiStatix^{™} technology available from Motorola Inc. USA or the 915MHz RFID Tag from Alien Technology, USA. Placing the vial close to the meter to load a strip using the strip loading means (not shown) provides the opportunity for the meter electronics to read or write to the contents of the proximity memory. This interaction with a vial's proximity is transparent to the user and does not require any additional actions by the user. The meter then reads the calibration code and any other relevant information from the proximity memory during the strip loading step. The memory 18 can contain anywhere from about 4 bytes up to 100 or more bytes of information. Typically, this memory would contain the calibration code value or values for a parametric equation transforming a raw glucose result into the reported glucose result. Other useful information that could be stored are expiration date of the vial, the number of strips that remain in the vial, the date of the first strip was removed from the vial (used for tracking vial-open expiration), lot code information etc. Memory 18 could also contain a CRC, CHECKSUM or other means of confirming the memory contents. This information can be used to increase the confidence of the glucose result and reduce the incidence of product misuse. In addition, information can be written to the proximity memory.

In addition, or alternatively, a 'clever' memory device incorporating re-writable memory such as an EEPROM and a microprocessor or microcontroller (typically a microprocessor with its own memory and appropriate port connections) may be used. Thus the re-writable memory device would have data storage and retrieval capabilities and local software function execution capabilities. Such a device would form re-writable memory device 18 and in addition to the READ/WRITE memory function would be able to undertake simple operations, or calculations. In particular such a device would aid upgrading of software, and in particular software associated with the user interface and/or the calculation algorithm and can allow an intelligent check of success of the upgrade. The 'clever' memory can be sent back to the manufacturer for verification of the status (success, failure etc. of the upgrade). Such devices include AT90SC9608RC (Secure microcontroller for smart cards) which has 96K Bytes of ROM program memory, 8K Bytes of EEPROM and 3K Bytes of RAM available from ATMEL of California USA, or the AE46C (smart card integrated circuit with 68KB EEPROM, 160KB ROM and 6KB RAM and a 1024-bit co-processor) available from Hitachi Ltd, Tokyo Japan, and Hitachi America Ltd, USA.

Figures 5 and 6 illustrate plan and side elevation views of an alternative sensor bank that can be used in a system in accordance with the invention. Here a sensor bank 10 is provided in the form of a substantially circular disc 10 comprising sensors 17A to 17K. Each sensor is physically connected to its neighbour during manufacture to form a disc, or the manufacturing process, may be used to lay down sensors such as strips on a substrate to from the disc. A smart chip 18 is fixedly connected to the base of the disc, for example, by adhesive.

A fluid sample such as blood or interstitial fluid, is placed in the entrance to capillary 32 for delivery to a test region 29 on the sensor. Each sensor can be used in turn. Notches or recesses 34, 36 and 38 can be used to aid the location of, a user's finger say, adjacent a sensor and thereby to facilitate delivery of a sample fluid to capillary channel 32. Notch 38 in particular provides a sharp corner, which can break the surface tension of a drop of fluid presented to it for aiding the ingress of fluid into the capillary 32. Such sensor banks can be screen printed, for example using appropriate ink such as carbon ink to provide conductive paths, insulation ink, reagent ink, for example containing an enzyme, and adhesive ink. Such sensor banks may also be produced in a flat bed process or in a continuous process of manufacture such as that described in International Patent Application PCT/US01/10097 publication number WO01/73109 entitled 'Continuous Process for the Manufacture of Disposable Electrochemical Sensors' filed on 28 March 2001 US Provisional Patent Application 60/436,683 filed on 27 December 2002 entitled 'Movable Flat Screen Printing in a Process for Manufacture of Disposable Electrochemical Sensors' and US Provisional Patent Application 60/422,230 'Preconditioning of the substrate in a continuous web process' filed on 30 October 2002.

In Figure 5, smart card 18 is fixed to sensor bank 10 by adhesive 23. Adhesive 23 may be cold cure adhesive, pressure sensitive adhesive, heat seal adhesive or hot melt adhesive. Substantially circular disc 10 has a central hole 25 which allows the device to be located within a meter. Smart chip 18 is asymmetrically located on the underside of disc 10. Smart card 18 may be symmetrically located about central hole 25 or connections for smart card 18 may be located symmetrically about central locating hole 25. Bank 10 may be rotatable within the meter or within a housing, to which the smart chip may be attached.

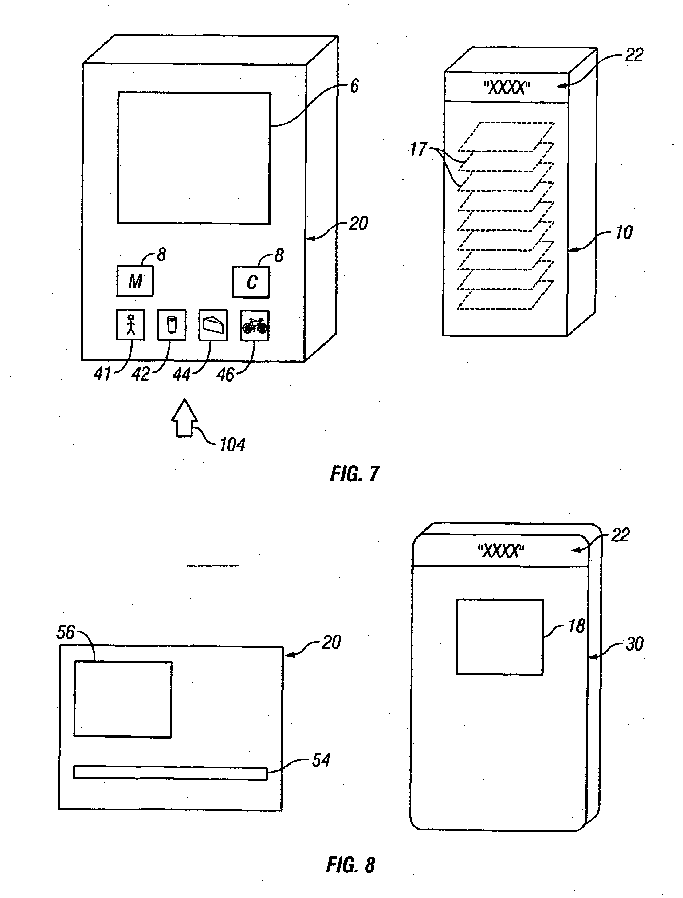

Figure 7 shows in perspective view a meter 20, a sensor bank in the form of a test strip cartridge 10 having a stack of test strips sensors 17 and a unique identifier code 'XXXX' 22. A separate smart chip 18 is provided mounted on a cardholder 30 typically, either smart chip 18 and smart chip cardholder 30 or both are provided with unique identifier code 'XXXX'. For example, code 22 when displayed on cardholder 30 may be in man readable form or indeed in machine-readable form such as in the form of a bar code. Alternatively or in addition, the identifier code may be provided in machine-readable form on smart chip 18. Smart chip 18 is attached to cardholder 30. Typically, cardholder 30 is the size and shape of a credit card i.e. around 55mm in width and around 87mm in length. Meter 20 is provided with a display 6 and several control buttons, which allow information concerning the activities of a user to be input straight away following depression of the control button. For example, button 41 allows information to be input about a patient's health, button 42 allows information to be inserted into the meter about a patient's medication, button 44 allows information to be inserted about a patient's food intake, button 46 allows information to be inserted about a patient's exercise. The remaining buttons 8 are used to switch the meter on and off and take a measurement.

Looking at meter 20 in the direction of arrow 104 a base view of meter 20 can be seen in Figure 8. Figure 8 shows a slot 56 for removable insertion of sensor bank 10 into meter 20. Typically a door (now shown) is provided. A slot 54 is provided for removable insertion of card 30 into meter 20. Optionally, a door (not shown) for closure of slot 54 is provided. Both bank 10 and card 30 carry unique identifier codes 'XXXX'. Whilst in this embodiment unique identifier code 22 is illustrated in human readable form, it may, as an alternative or in addition be in machine-readable form separate and distinct from smart chip 18. As an alternative or in addition smart chip 18 can contain this unique identifier code in machine-readable form.

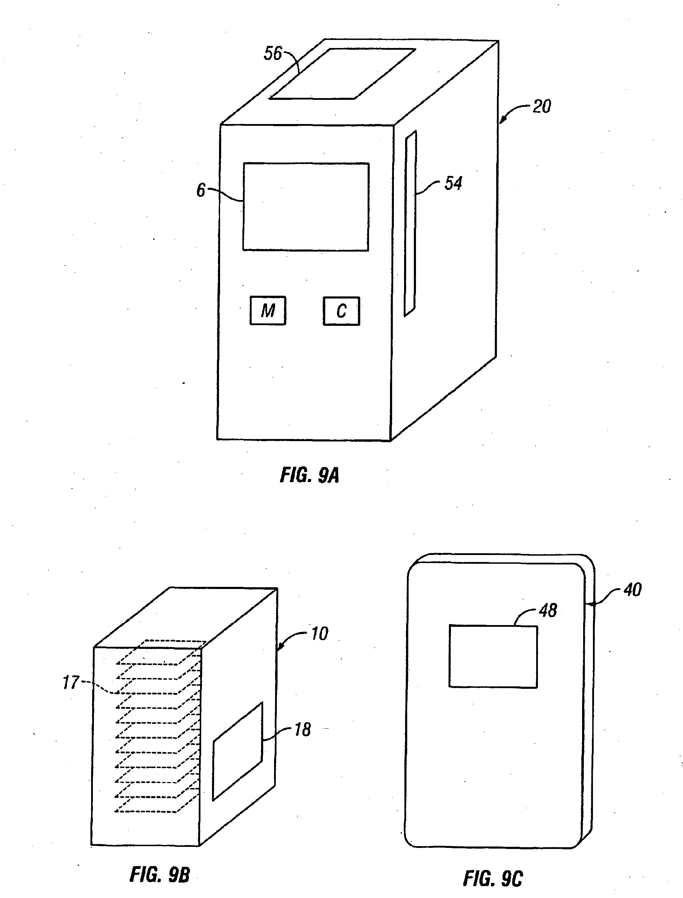

Figures 9A, 9B and 9C illustrate perspective views of a meter 20, strip bank 10 and credit card sized smart chip carrier 40. In this embodiment that can be used in a system of the invention two re-writable memory devices, here in the form of smart chips, are provided. One smart chip is physically fixedly attached to the housing of strip bank 10 so as to be uniquely associated with test strips 17 contained within the housing of strip bank 10 in a physical manner, and a second smart chip 48 attached to a separate, in this case credit card sized, card carrier 40. Slots 54 and 56 are sized and shaped to receive respectively credit card sized smart chip carrier 40 and strip cartridge 10 removably within meter 20. The provision of two smart chips one physically linked to the strip cartridge and a second one on a separate removable card carrier, has certain advantages as will be described in more detail later.

Many of the advantages of the present invention result from linking in a quasi-permanent manner at least two and typically a set of test strips for measuring an analyte in a fluid, such as a physiological sample, to a re-writable memory device such as a smart chip.

The link may be physical or wireless, but typically it creates an almost unbreakable association between information contained on the memory device and a set of test sensors. Thus if a smart chip 18 were broken from the side of vial 10 the vial would to all intents and purposes be unusable. Also, if a vial 10 with one unique identifier is used and a smart chip 18 with another unique identifier is used, testing with meter 20 is typically prevented. Thus during manufacture, quasi-permanent association is formed between a set of test sensors and a re-writable memory device. Indeed individual test sensors, such as test strips, may each be provided with the unique identifier of the bank. This can provide extra security in the association between re-writable memory, sensor bank and sensors, particularly useful in wireless arrangements. This association between test sensors and information exists for information to be uploaded to the meter from the re-writable memory device and information to be downloaded to the re-writable memory. For example, information relating to variations in manufacture, which can result in loss of precision in the results from test strips, can be loaded into the smart chip and read by the meter to be used in connection with those strips to which that information relates. Thus, the relationship between the information and the test strips is determined, during manufacture, in a controlled and regulated manner and carried through to a test and handling of test result. A substantially secure system is provided by the invention in which information relating to test strips can be carried to and from the microprocessor of the meter via a smart chip clearly and typically uniquely associated with those test strips. Clearly this can be achieved in a physical manner in which the smart chip is fixedly mounted to a strip bank, for example to the housing of a sensor bank or to a set of sensors. Alternatively, this can be achieved in a wireless manner to a slightly less secure extent by the provision of unique identifier codes within the sensor bank and within the re-writable memory device carrier or indeed within the re-writable memory device as in the embodiments shown in Figure 1B.

Physically connecting the smart chip to the strip bank further reduces the risk of the wrong smart chip and hence incorrect information, such as calibration code, being inadvertently associated with a set of strips. This is particularly true in the case where the strip bank contains a set of loose strips, for example, a vial of strips, rather than a cartridge in which strips are delivered on a one-by-one basis. The risk of the smart chip being associated with the wrong test strips is further reduced if the strip bank is physically connected to or loaded into the meter and a means for delivering test strips is provided for delivering strips one by one from the meter. The risks of the wrong information on a smart chip being associated with a set of test strips is even further reduced when, in the scenario just described, a means for delivering the test strip cartridge to a test strip port is provided within the meter. Thus, once the test strip cartridge is inserted into the meter, the user is not allowed access to the test strip cartridge. Therefore the meter microprocessor accesses the smart chip associated with that set of test strips in that test strip container and that information is used to convert the raw data from the test strip to a glucose measurement to be seen by a patient on the display 6. Thus the system of the invention is particularly secure in the example and embodiment in which a set of test strips is provided in the form of a strip bank and a smart chip is physically attached to the strip bank, the strip bank being insertable into the meter 20 and thereafter strips being delivered to a test port ready for testing by application of a sample, typically blood. Furthermore, information from test results for those strips or updated strip count can be written back to the smart chip associated with those strips.

The invention finds particular application in testing of a concentration of glucose in blood although other applications of the invention can be envisaged from the description contained herein, for example, for detection of glucose or fructosamine in blood or interstitial fluid or of glucose in urine of haemoglobin in blood, of other hormones in blood or ISF or urine or other analytes in other physiological fluids.

Whilst some embodiments of the invention have only one smart chip 18 associated either physically or wirelessly with a sensor bank 10, other embodiments envisage the use of two smart chips. Where one re-writable memory device such as a smart chip is used, this smart chip and typically its carrier or container of strips to which it is attached can be carried to a smart card reader for download to a personal computer whether at home or in a clinician's office. Thus, information concerning testing and a patient can be simply and easily transported to a memory reader for analysis, with little additional cost.

The embodiment shown in Figure 9 utilises a first re-writable memory device such as smart chip 18 physically attached to the sensor bank 10 and a second re-writable memory device such as smart chip 48 attached to a separate card carrier 40. Thus, test results from microprocessor contained within meter 20 can be stored within first smart chip 18 or within second smart chip 48. Indeed, whilst typical sizes of the smart chip can be 1KB or more it is envisaged that the second smart chip 48 will have a greater capacity than the first smart chip 18. Thus, either smart chip could be used to carry results to a smart chip reader or download to a personal computer or the Internet. Indeed either smart chip may be transported by a user to a physician's office where the smart chip could be read by a smart card reader and downloaded to a physician's personal computer. Thus, the invention provides a method of transporting data using a smart chip.

Affixing the smart chip to the side of the strip bank provides certain advantages in terms of reducing the risk of mixing up the information to be uploaded to the meter such as the calibration code (to be used during testing of strips), or for information to be downloaded from the meter to the smart chip (such as the test results from that batch of tests), and ensuring information handling, transportation and association with a set of strips is more secure. However, the shape of a stack of strips and the strip cartridge 10 is a little cumbersome to carry around. Thus, patients may prefer the use of a strip bank 10 with a re-writable memory device attached such as that shown in Figure 5 or the separate card carrier 30 seen in Figure 7.

Since a sensor bank such as that illustrated in Figure 5 may be considered a bio-hazard once a number of tests have been conducted, a pouch or other container (not shown) may be provided for insertion of the sensor bank thereinto for later transport to a physician's office or other place which may have a re-writable memory reader for reading said re-writable memory device. The embodiment shown in Figure 9 combines the advantages of a smart chip physically fixedly attached to a sensor bank 10 and a separate conveniently sized smart chip card carrier. The carrier shown in this example is the size and shape of a credit card 40. Furthermore, provision of a second chip carrier allows for the use of differently sized memorys and hence differently priced smart chips. Thus, although smart chip 18 may be sufficiently sized to carry the last six months worth of testing or indeed one year's testing and also all associated data as hereinbefore described such as calibration codes, lot codes, container open life at test, health, food, exercise status of patient and so on, smart chip 48 may be larger and more costly particularly if it is to be used for a longer period of time by an individual. Typically re-writable memory device 18 will be disposed of after the strips within it are used up and the data from the smart chip has been downloaded to an appropriate data storage/analysis device, if desired. However, card 40 and re-writable memory device 48 is capable of being used for a longer of period of time say for 50 sets of strips within a strip bank 10 or over several months or years thus, smart chip carrier 40 may be a patient-specific, personalised account of test data which is sized and shaped to be conveniently carried around, for example credit card sized to fit in a wallet. Of course, other sizes of smart card carriers 40 may be considered as lying within the scope of this invention.

A further advantage of the use of a re-writable memory device associated with a set of strips is that information related to software upgrades or corrections can be carried on the re-writable memory device. In particular, a dummy sensor bank 10 could be provided carrying a re-writable memory device of greater than usual capacity for uploading software upgrades and in particular user interface software of algorithm calculation software or corrections or personalisation of software to the meter. In this case the dummy sensor bank would not contain any sensors or contain only dummy sensors. Children in particular will benefit from personalised software, which communicates with them in a manner suitable for their age. Alternatively, real or even dummy sensor banks 10 could be provided with re-writable memory device such as smart chips which have country or language data to flavour a meter for a particular country or language or indeed to provide updated information to a patient. Thus, the size (and hence cost) of the re-writable memory device can be varied to suit the particular application required.

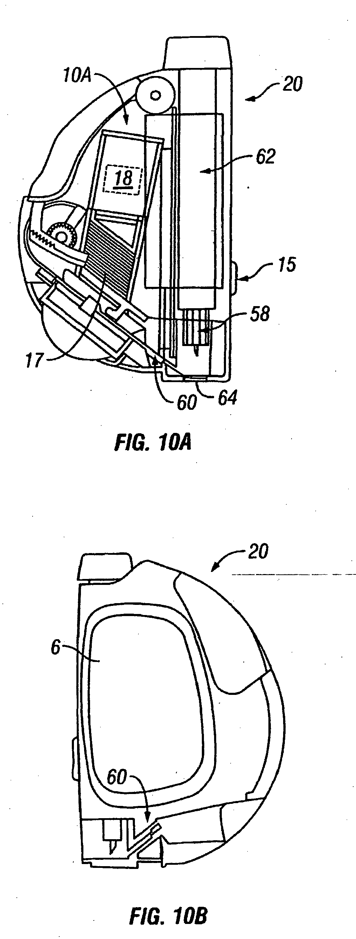

Figure 10A shows a combined lancet and meter 20 having a display 6, lancet 58 and strip delivery chute 60.

Figure 10B illustrates a cut-away view of the combined meter and lancet 20 of Figure 10 incorporating a cartridge of stacked strips 10A. A lancet comprising a lancet drive train 62 and a lancet 58 is provided. Lancet 58 is launched by drive train 62 via aperture 64 into a users skin to draw blood. A strip is delivered via chute 62 to a position adjacent aperture 64 and a drop of blood, which typically forms following lancing. Strip cartridge 10A contains a stack of strips 17 at an oblique angle to the housing of cartridge 10A. A smart chip 18 is glued to the side of cartridge 10A.

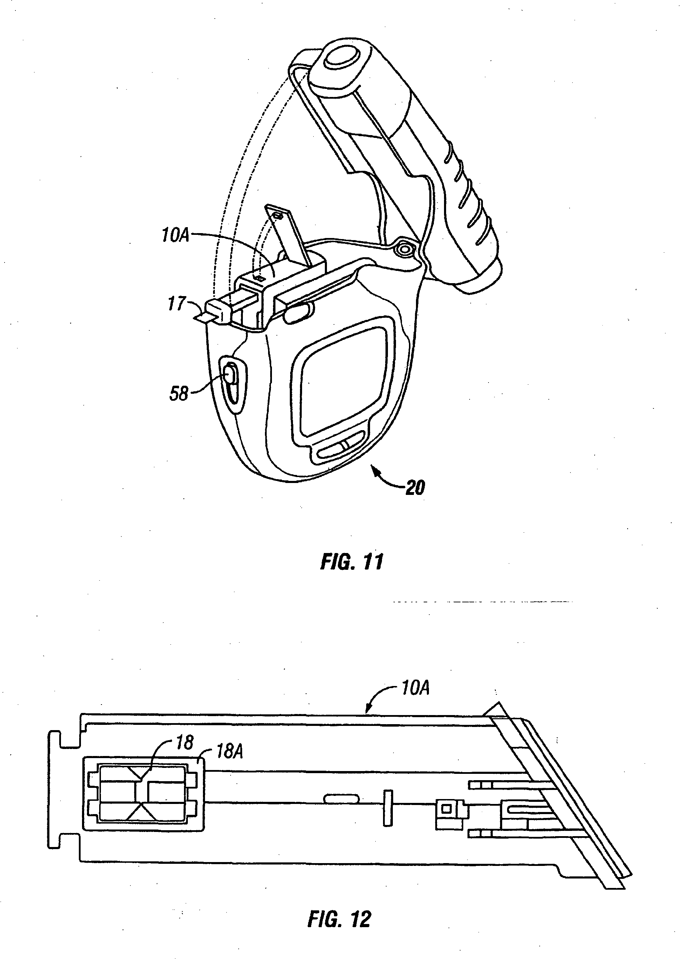

Figure 11 illustrates another example of a meter 20 incorporating a strip dispenser having a smart chip 18 fixedly attached hereto.

Figure 12 shows in side elevation view a close up of one side of strip cartridge 10A showing a smart chip 18 located in a matchingly sized recess 18A on the side of housing 10A. Smart chip 18 is typically glued by an adhesive to the side of cartridge 10A. The housing of cartridge 10A is formed from polypropylene, which has low moisture vapour transmission characteristics useful in preventing the ingress of moisture to test strips 17.

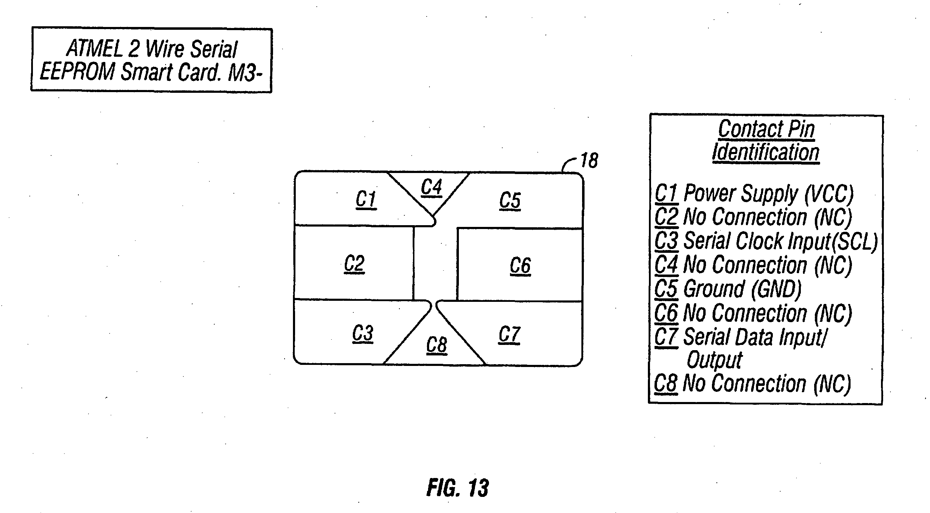

Figure 13 shows in more detail an example smart chip 18 in this case an EEPROM smart card from Atmel and the contact pin identification details.

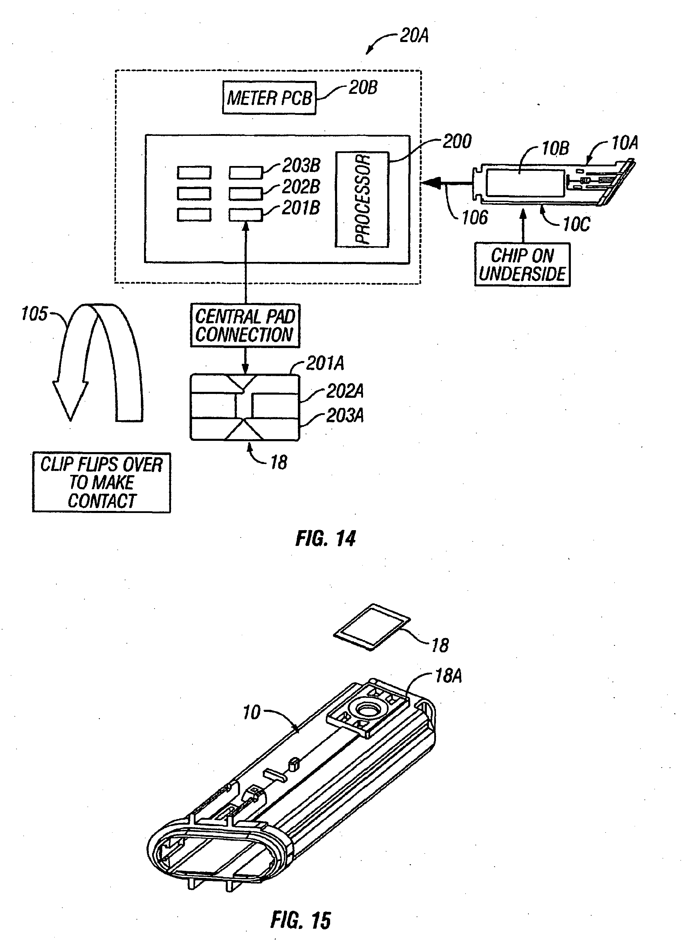

Figure 14 shows a schematic diagram indicating a meter 20A having a printed circuit board 20B and microprocessor 20C. Arrow 106 indicates the direction of the insertion of a strip cartridge 10A into meter 20A. A panel 10B is provided on one side of cartridge 10A for attaching or printing a man-readable code thereon. Smart chip 18 is located on the rear side 10C of cartridge 10A. Smart chip 18 is rotated in the direction of arrow 105 to make contact to pins within meter 20A. Thus, pins 201 A, 202A and 203A on chip 18 connect with pads 201B, 202B and 203B in meter 20A.

Figure 15 shows a perspective view of a strip cartridge 10 and smart chip 18 and in particular shows in more detail the recess 18A within which smart chip 18 is located on the housing of strip cartridge 10. Typical glue such as a pressure sensitive, heat seal or hot melt adhesive is used to attach the smart chip to the housing of cartridge 10.

Typically, the re-writable memory is a smart chip in the format of an EEPROM (electronically erasable programmable read only memory.) Such a device does not require power to retain the contents of its memory.

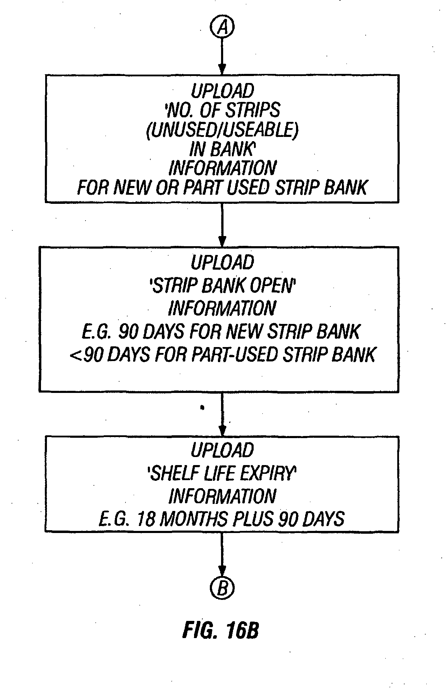

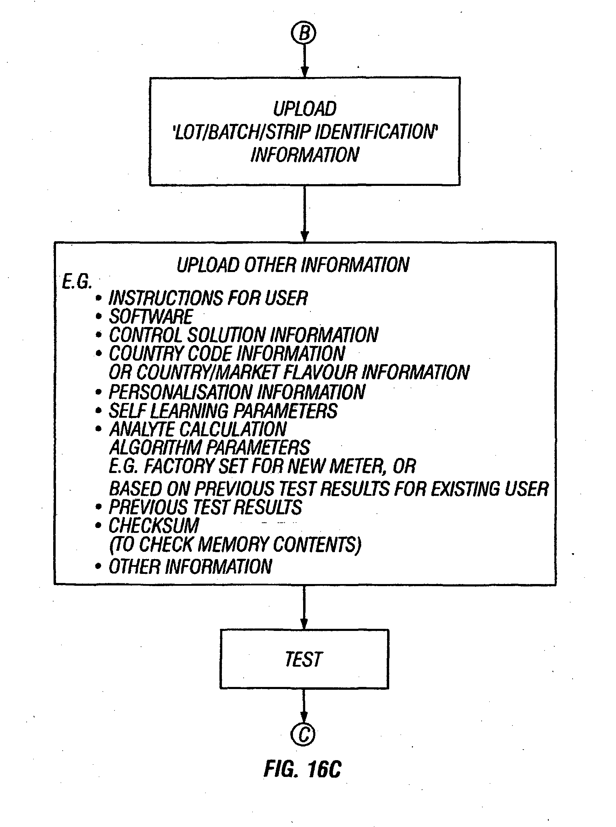

Figure 16A, 16B and 16C illustrate a series of steps, any of which may be optional, as will be understood by those more skilled in the art, which might be followed on insertion or connection of a strip bank 10 into a meter 20. It will be understood by those skilled in the art from the information contained herein that one or more items of information is typically uploaded from the strip bank smart chip into the meter. Typically this information will be the calibration code, thus rendering the step of calibrating the meter to a new batch of strips, transparent to a user. If a strip bank is removed or disconnected from the meter when only some of the strips have been used, then a useful application of the present invention is downloading of the number of strips remaining in the strip bank to the memory device. On re-connection of the part-used bank the number of strips remaining can be uploaded from the associated re-writable memory. Other possibilities include the use of a strip bank in which only some of the strips are usable. These strips can be located and used if information concerning the location and type of usable strips is contained within the smart chip. Thus, an otherwise unusable set of strips in a strip bank might be usable if re-writable memory device can be used to identify the location of those usable strips to the meter. Such an arrangement might find particular application in a sensor bank such as that shown in Figure 5 and 6.

Figure 17 shows the type of information that might be downloaded to a sensor bank on completion of a test and also the steps that might be undertaken during a test. The point at which the steps of Fig. 16A to C connect with the steps of Fig. 17 is seen at 'C'. It will be understood by those skilled in the art from the information contained herein that one or more items of information can be downloaded from the meter to the strip bank re-writable memory device on completion of a test or just prior to switch off.

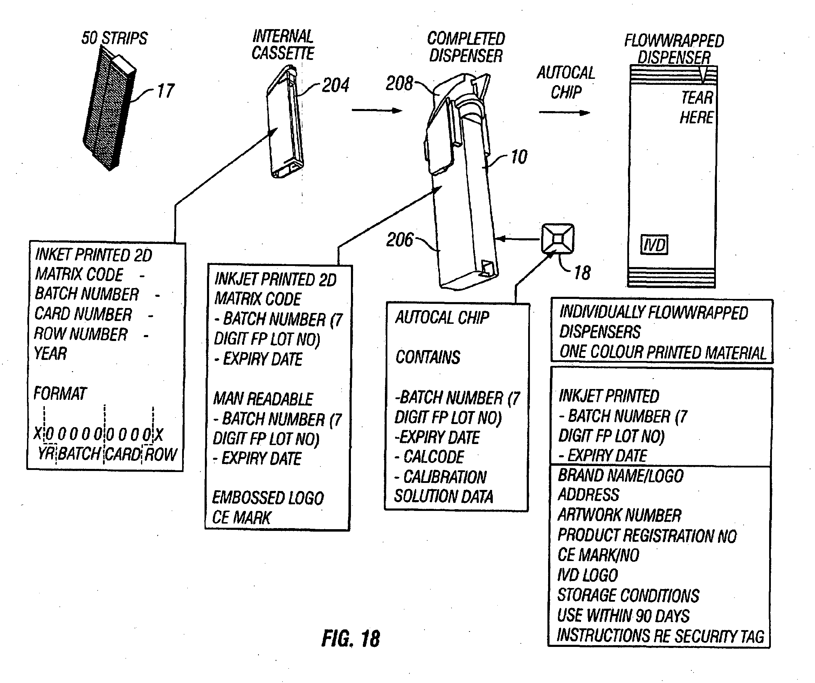

Figure 18 shows a schematic diagram indicating the type of information which may be affixed to the various levels of packaging of a strip bank 10. A set of fifty strips 17 are loaded into an internal cassette 204 before being loaded into a completed dispenser 10 having a main portion 206 and a lid portion 208. Information such as a 2D matrix code detailing batch number, code number, row number and year is printed onto the outside of the internal cassette 204. Once internal cassette 204 is loaded into the cartridge housing 206 a 2D matrix code is printed on the outside indicating the batch number and expiry date. In addition, a human-readable code is printed detailing the batch number and expiry date. In addition, an auto-calibration smart chip 18 is affixed to the rear channel within the re-writable memory of which is contained, a batch number, expiry date, calibration code and control or calibration solution data. Auto-calibration is the uploading of calibration code automatically to the meter with minimal user intervention. Finally, the strip bank 10 is wrapped ready for sale. The outside of the flow wrap (which may be a foil wrap) is printed with batch number, expiry date and other identifying details for branding purposes.

Figure 19 details the type of information which might be uploaded from a re-writable memory device to the meter and the type of information which might be written back down from the meter to the re-writable memory device for later use by a patient or clinician, or for use during further testing. The software of meters in the field may need to be upgraded and this invention can be used to fix at least three types of changes. These are 'corrective' -to fix problems, 'adaptive' -to change the software in the light of changes to the environment in which the software runs (e.g. regulatory changes) and 'perfective' -to change the software to add new features. The invention also provides a method of dynamically flavouring the meter with country code, personalised or country flavoured software, software upgrades and parameters related to previous test results for updating of the testing algorithm for future tests. Furthermore the invention provides a method of downloading test results within a clinician's office by removing the strip bank with re-writable memory device, such as an associated smart card and carrying this to a clinician's office or to a home personal computer for downloading to a software application. Furthermore the invention provides a method of clinical testing to record test results and a method of updating parameters based on previous test results. One advantage of the method of the present invention is that the available space in re-writable memory device can far exceed what might normally be available on a microprocessor in a meter and thus test results for extended periods of time can be measured and in addition further data can be stored in conjunction with said test results such as health, food, exercise, data relating to the patient, time of test, time of test container open, time of test relative to expiry date and so on providing detailed clinical data. Furthermore, the invention provides a method of linking either physically or wirelessly strips to a re-writable memory device carrying calibration data. Furthermore the invention provides the ability to use dummy sensor banks such as dummy dispensers or strip banks, which carry re-writable memory devices having extra memory or extra components such as microprocessors or microcontrollers, for example, for carrying software upgrades. The invention also provides for a smart card reader which is adapted to receive a strip bank to read the information contained on the re-writable memory device of the strip bank. Further the invention provides a system using a sensor bank having re-writable memory device including re-writable memory and optionally, a microprocessor and/or proximity components for providing 'clever' memory and/or 'proximity' memory associated with a set of sensors.

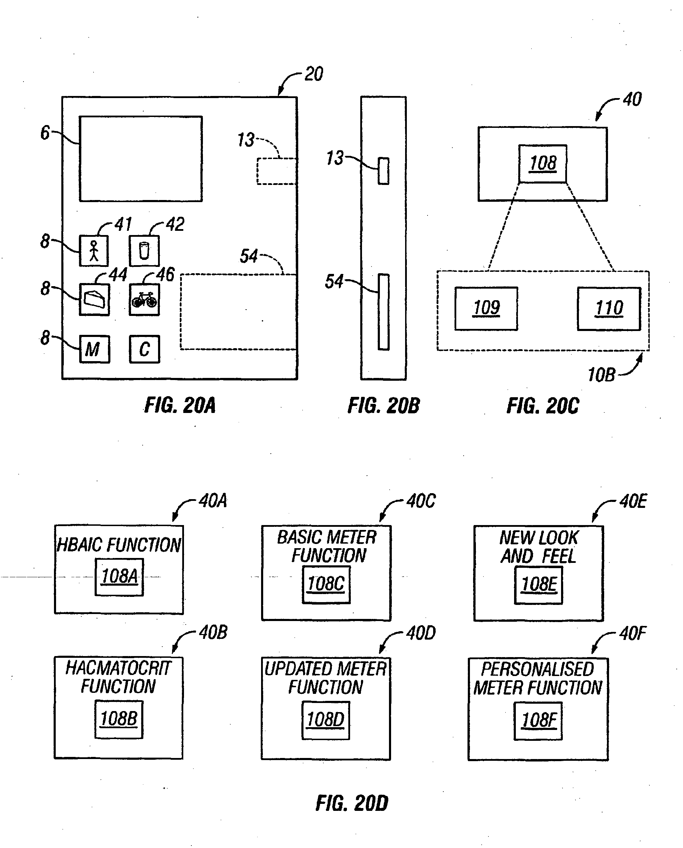

In yet a further aspect the invention provides a system comprising a meter such as meter 20 and a re-writable memory device having re-writable memory and a microprocessor, for example, as herein before described. One example of this is a credit-card sized smart card carrier 40 and meter 20, the smart card comprising a combined re-writable memory and integrated circuit. In the broadest sense in this aspect, there would be no strip bank. Although one embodiment of this aspect may include a strip bank with a re-writable memory device, which, optionally, may consist of 'clever' memory, having in addition a microprocessor. An example of the components of such a system is shown in Figures 20A and 20D as will be described later.

The two-way nature of the information exchanged between the meter and the re-writable memory device provides much of the additional functionality described above. Whilst the advantages of the invention are provided by the embodiment in which the re-writable memory device is wirelessly connected to the strip bank, permanently attaching the re-writable memory device to the strip bank, whether this is a container, dispenser, substrate with sensors thereon, cassette or the like, provides many advantages since the risk of mixing up the test sensors with incorrect information on another re-writable memory device are significantly reduced. Furthermore, in those embodiments in which the strip bank is linked to the meter (so that delivery of a strip ready for testing is outside the control of a patient and, optionally, the patient cannot independently remove the strip without triggering a write to the re-writable memory indicating that the strip has been removed and therefore a test has been taken) is advantageous. If the strip bank is inside the meter, and a strip delivery means is provided to deliver the strip to a strip port ready for a testing, with no patient access to sensors or re-writable memory once the strip bank is placed inside the meter, then there is a virtual guarantee that the sensors used in subsequent tests came from that bank. Furthermore, downloading the number of strips remaining to the strip bank smart chip on switch off of the meter allows the user to remove a partially-used strip bank and insert another one, for example, a full one, for example for travelling. On his return, the user can insert the part-used bank and the number of strips remaining to be used is uploaded via the information link to the meter. Furthermore, strip bank open information detailing when the strip bank was opened can be uploaded. A check is then made in the meter to see if this lies within the recommended length of opening of the strip bank. If the software of the meter thus allows, a warning to a user concerning further use of that strip bank is given.

Alternative embodiments and advantages of the invention will be envisaged by those skilled in the art from the information contained herein. All such embodiments considered to lie within the scope of this invention as defined by the attached claims.

Figure 20A illustrates a meter 20 having a display 6 and control buttons 8 some of which, namely 41, 42, 44 and 46 allow direct input of information concerning respectively health, medication, food intake and exercise. A strip port 13 is provided for inserting a test strip into as has been herein before described. Slot 54 in housing of meter 20 is sized shaped and adapted to receive and adapted to connect to a smart card carrier 40 in which a re-writable memory device typically in the form of a smart chip 108 is provided. Device 40 and smart chip 108 are shown in more detail in Figure 20C. In particular, smart chip 108, may comprise two components. The first component 109 consists of re-writable memory, and the second component 110 consists of an integrated circuit or microprocessor. Thus, re-writable memory device 108 is 'clever' memory as has been described previously. The meter may or may not contain its own microprocessor for control of the test measurement, algorithm calculation, user interface, including display and so on. A smart card 40 with 'clever' memory 108 slotted into slot 54 connecting to the meter can provide some or all of this functionality. For example, a microprocessor and associated circuitry may be provided within the meter for driving the display and control buttons whilst the user interface software, test measurement software and algorithm calculation software may be provided on the smart card 40 so that upon insertion of smart card 40 into slot 54 of meter 20, meter 20 is a fully functional meter. The re-writable memory device 108 will typically contain RAM as well as EEPROM 109 and other associated circuitry for storing and running part or all of the meter functionality. An alternative embodiment is one in which all of the meter functionality is already contained within a microprocessor within the meter and smart card 40 provides an update to that software or it may provide an additional function not present on the basic meter functionality.

Figure 20D illustrates six examples of smart cards which might be used in the system of Figures 2A-2C. Smart card 40A with smart chip 108A contains a HBA1C function software which can be used to supplement the basic meter functions already present in the meter. Smart card 40B contains information concerning a haematocrit function on smart chip 108B. Smart card 40C contains smart chip 108C housing basic meter functionality such as display drivers, button control drivers, user interface software, test measurement software, algorithm calculation software etc. Smart card 40D contains updated basic meter functionality on smart chip 108D in which at least one updated segment such as the user interface software, for example, is provided. Thus, the invention allows very easy field upgradability of meter software since the basic meter functionality is controlled within the replaceable or plug-able smart card 40. Of course the smart card 40 may simply be used for software upgrades rather than delivery of the basic meter functionality. Further examples include a smart card 40E which contains software having a new look and feel to be added to the meter functionality already present in the meter. Smart card 40F contains personalised meter functions for a particular user and may indeed include a detailed patient record as has already been described including, for example, previous test results, activities, food intake and trends and so on. Alternative embodiments of this aspect of the invention can be envisaged from the disclosure contained herein by those skilled in the art. All such embodiments are intended to lie within the scope of this invention.

## Claims

1. A system (2B) for conducting a test for an analyte in a physiological fluid comprising:
a test sensor bank (1B, 10) including more than one test sensor (17), at least initially, for testing for an analyte;
a re-writable memory device (1C, 18) associated with said test sensor bank;
a meter (1A, 20) for conducting a test using a test sensor from the bank;
the meter comprising reading means for reading information from the re-writable memory device and a writing means for writing information to the re-writable memory device; and
transmission means (100B, 102B) for transmitting information between the re-writable memory and the meter,
**characterised in that** the test sensor bank and the re-writable memory device each comprise a unique identifier code ('XXXX'), wherein the meter is configured to exchange information with the re-writable memory device only if the meter identifies that the re-writable memory device and the test sensor bank have a common unique identifier code.

2. A system according to claim 1 in which the re-writable memory device (1C, 18) is uniquely and/or permanently associated with the test sensor bank (1B, 10).

3. A system according to claim 1 or 2 in which the re-writable memory device (1C, 18) is physically attached to the test sensor bank (1B, 10).

4. A system according to claim 3 in which the re-writable memory device (1C, 18) is fixedly attached to the test sensor bank (1B, 10).

5. A system according to claim 4 in which the test sensor bank comprises a housing or substrate and the re-writable memory device (1C, 18) is adhered to the housing or substrate using adhesive.

6. A system according to claim 4 or 5 in which re-writable memory device (1C, 18) is adhered using a cold sure adhesive, heat seal adhesive, pressure sensitive adhesive, hot melt adhesive.

7. A system according to claim 5 or 6 in which the housing comprises a plastic such as polypropylene or polypropylene based plastic.

8. A system according to claim 5 or 6 in which the substrate comprises a plastic such as polyester or polypropylene based plastic.

9. A system according to claim 1 in which the re-writable memory device (1C, 18) is wirelessly attached to the test sensor bank (1B, 10) using RF (radio frequency) transmission.

10. A system according to claim 9 in which means are provided on the test sensor bank (1B, 10) and on the re-writable memory (1C, 18) for transmitting the unique identifier code ('XXXX') to the meter (1A, 20).

11. A system according to any preceding claim in which the test sensor bank (1B, 10) is removably physically connectable to the meter (1A, 20).

12. A system according to claim 11 in which the test sensor bank (1B, 10) is at least partially insertable into the meter (1A, 20).

13. A system according to any preceding claim in which the test sensor bank (1B, 10) comprises a stack of two or more sensors.

14. A system according to any of claims 1 to 12, in which the test sensor bank (1B, 10) comprises a card of two or more sensors.

15. A system according to any preceding claim in which said re-writable memory device is a first re-writable memory device (18) and a second re-writable memory device (48) is provided.

16. A system according to claim 15 in which at least one of the first and second re-writable memory devices (18, 48) is mounted on a card (30, 40).

17. A system according to claim 16 in which the card (30, 40) is substantially the size and shape of a credit card.

18. A system according to claim 5 in which the substrate is a substantially circular shape.

19. A system according to any preceding claim in which the test sensors are test strips (17).

20. A system according to any preceding claim in which the test sensors are screenprinted.

21. A system according to any preceding claim in which the meter comprises means for conducting a test.

22. A system according to any preceding claim in which at least one re-writable memory device (1C, 18) comprises an EEPROM or smart chip.

23. A system according to any preceding claim in which at least one re-writable memory comprises a microprocessor or microcontroller.

24. A system according to any preceding claim in which at least one re-writable memory device comprises a proximity memory.

25. A system according to any of claims 22 to 24 in which the memory device has a memory size of no less than 1K Bit, for example, selected from the group of 1K Bits, 2K Bits, 4K Bits, 8K Bits, 16K Bits, 32K Bits, 64K Bits, 128K Bits, 256K Bits, 512K Bits, 1024K Bits.

26. A system according to any preceding claim in which the analyte is glucose.

27. A system according to any preceding claim in which at least one piece of information selected from the group of:
Calibration Code
Number of unused or usable sensors
Sensor Bank Open (when the sensor bank was opened)
Shelf Life Expiry
Instructions for User
Software, corrections or upgrades
Country Code
Country or Market Flavour Information
Personalisation Information
Self-Learning Parameters
Upgraded/Updated analyte calculation Algorithm parameters
Previous Test Results
CHECKSUM (checking the memory contents)
Parameters for User Interface
Information about New Products
Information about Performance of Product
is uploadable from said re-writable memory device into the meter.

28. A system according to any preceding claim in which at least one piece of information selected from the group of:
Calibration Code
Number of unused or usable sensors
Sensor bank Open (when the sensor bank was opened)
Shelf Life Expiry
Instructions for User
Software, corrections or upgrades
Country Code
Country or Market Flavour Information Personalisation Information
Self-Learning Parameters Upgraded/Updated analyte calculation parameters
Previous Test Results
CHECKSUM (checking the memory contents)
Parameters for User Interface
Information about New Products
Information about Performance of Product
is downloadable to said re-writable memory device.

29. A system according to any preceding claim in which at least one piece of information selected from the group of:
Calibration Code
Number of unused or usable sensors
Sensor bank Open (when the sensor bank was opened)
Shelf Life Expiry
Instructions for User
Software, corrections or upgrades
Country Code
Country or Market Flavour Information
Personalisation Information
Self-Learning Parameters Upgraded/Updated analyte calculation Algorithm parameters
Previous Test Results
CHECKSUM (checking the memory contents)
Parameters for User Interface
Information about New Products
Information about Performance of Product
is uploadable or downloadable to a second re-writable memory device (48).

30. A method for conducting a test for an analyte in a physiological fluid comprising providing a system (2B) according to any of claims 1 to 29; using the system to conduct a test for an analyte in a physiological fluid; and uploading at least one piece of information selected from the group of:
Calibration Code
Number of unused or usable sensors
Sensor bank Open (when the sensor bank was opened)
Shelf Life Expiry
Instructions for User
Software, corrections or upgrades
Country Code
Country or Market Flavour Information
Personalisation Information
Self-Learning Parameters Upgraded/Updated analyte calculation Algorithm parameters
Previous Test Results
CHECKSUM (checking the memory contents)
Parameters for User Interface
Information about New Products
Information about Performance of Product
from at least said first re-writable memory device (1C, 18) onto the meter (1A, 20).

31. A method according to claim 30 comprising downloading, from the meter (1A, 20), at least one piece of information selected from the group of:
Calibration Code
Number of unused or usable sensors
Sensor bank Open (when the sensor bank was opened)
Shelf Life Expiry
Instructions for User
Software, corrections or upgrades
Country Code
Country or Market Flavour Information
Personalisation Information
Self-Learning Parameters
Upgraded/Updated analyte calculation Algorithm parameters Previous Test Results
CHECKSUM (checking the memory contents)
Parameters for User Interface
Information about New Products
Information about Performance of Product
to at least said first re-writable memory device (1C, 18).

32. A method for conducting a test for an analyte in a physiological fluid comprising providing a system according to any of claims 1 to 29; using the system to conduct a test for an analyte in a physiological fluid; and uploading at least one piece of information selected from the group of:
Calibration Code
Number of unused or usable sensors
Sensor bank Open (when the sensor bank was opened) Shelf Life Expiry
Instructions for User
Software, corrections or upgrades
Country Code
Country or Market Flavour Information
Personalisation Information
Self-Learning Parameters
Upgraded/Updated analyte calculation Algorithm parameters Previous Test Results
CHECKSUM (checking the memory contents)
Parameters for User Interface
Information about New Products
Information about Performance of Product
from at least said second re-writable memory device (48) onto the meter (1A, 20).

33. A method according to claim 32 comprising downloading, from the meter (1A, 20), at least one piece of information selected from the group of:
Calibration Code
Number of unused or usable sensors
Sensor bank Open (when the sensor bank was opened)
Shelf Life Expiry
Instructions for User
Software, corrections or upgrades
Country Code
Country or Market Flavour Information
Personalisation Information
Self-Learning Parameters Upgraded/Updated analyte calculation Algorithm parameters
Previous Test Results
CHECKSUM (checking the memory contents)
Parameters for User Interface
Information about New Products
Information about Performance of Product
to at least said second re-writable memory device (48).

34. A method according to any of claims 30 to 33 wherein a test sensor being used for a test remains within the sensor bank (1B, 10) during a test.

35. A method according to any of claims 30 to 34 wherein a test sensor is at least partially removed from the sensor bank for use during a test.

36. A method according to claim 35 comprising delivering a sensor to a test port for a test.

37. A method according to any of claims 30 to 36 comprising measuring the concentration of an analyte.

## Patentansprüche

1. System (2B) zum Durchführen einer Untersuchung auf ein Analyt in einem physiologischen Fluid, das Folgendes umfasst:
eine Testsensorbank (1B, 10), die zumindest anfänglich mehr als einen Testsensor (17) für die Untersuchung auf ein Analyt enthält;
eine wiederbeschreibbare Speichervorrichtung (1C, 18), die der Testsensorbank zugeordnet ist;
ein Messgerät (1A, 20) zum Durchführen einer Untersuchung unter Verwendung eines Testsensors von der Bank;
wobei das Messgerät Lesemittel zum Lesen von Informationen von der wiederbeschreibbaren Speichervorrichtung und Schreibmittel zum Schreiben von Informationen auf die wiederbeschreibbare Schreibvorrichtung umfasst; und
Übertragungsmittel (100B, 102B) zum Übertragen von Informationen zwischen dem wiederbeschreibbaren Speicher und dem Messgerät,
**dadurch gekennzeichnet, dass** die Testsensorbank und die wiederbeschreibbare Speichervorrichtung beide einen eindeutigen Identifikationscode ('XXXX') umfassen, wobei das Messgerät konfiguriert ist, Informationen mit der wiederbeschreibbaren Speichervorrichtung nur auszutauschen, wenn das Messgerät ermittelt, dass die wiederbeschreibbare Speichervorrichtung und die Testsensorbank einen gemeinsamen eindeutigen Identifikationscode besitzen.

2. System nach Anspruch 1, wobei die wiederbeschreibbare Speichervorrichtung (1C, 18) einmalig und/oder dauerhaft der Testsensorbank (1B, 10) zugeordnet ist.

3. System nach Anspruch 1 oder 2, wobei die wiederbeschreibbare Speichervorrichtung (1C, 18) an der Testsensorbank (1B, 10) physikalisch befestigt ist.

4. System nach Anspruch 3, wobei die wiederbeschreibbare Speichervorrichtung (1C, 18) fest an der Testsensorbank (1B, 10) befestigt ist.

5. System nach Anspruch 4, wobei die Testsensorbank ein Gehäuse oder ein Substrat umfasst und die wiederbeschreibbare Speichervorrichtung (1C, 18) an das Gehäuse oder das Substrat unter Verwendung von Klebstoff angeklebt ist.

6. System nach Anspruch 4 oder 5, wobei die wiederbeschreibbare Speichervorrichtung (1C, 18) unter Verwendung eines kältesicheren Klebstoffes, eines Heißsiegelklebstoffes, eines druckempfindlichen Klebstoffes, Schmelzklebstoffes angeklebt ist.

7. System nach Anspruch 5 oder 6, wobei das Gehäuse einen Kunststoff wie etwa Polypropylen oder Kunststoff auf Polypropylenbasis umfasst.

8. System nach Anspruch 5 oder 6, wobei das Substrat einen Kunststoff wie etwa Polyester oder Kunststoff auf Polypropylenbasis umfasst.

9. System nach Anspruch 1, wobei die wiederbeschreibbare Speichervorrichtung (1C, 18) unter Verwendung von Hochfrequenzübertragung (HF-Übertragung) an die Testsensorbank (1B, 10) drahtlos angeschlossen ist.

10. System nach Anspruch 9, wobei Mittel auf der Testsensorbank (1B, 10) und auf dem wiederbeschreibbaren Speicher (1C, 18) vorgesehen sind, um den eindeutigen Identifikationscode ('XXX') an das Messgerät (1A, 20) zu senden.

11. System nach einem der vorhergehenden Ansprüche, wobei die Testsensorbank (1B, 10) mit dem Messgerät (1A, 20) abnehmbar physikalisch verbindbar ist.

12. System nach Anspruch 11, wobei die Testsensorbank (1B, 10) zumindest teilweise in das Messgerät (1A, 20) einfügbar ist.

13. System nach einem der vorhergehenden Ansprüche, wobei die Testsensorbank (1B, 10) einen Stapel von zwei oder mehr Sensoren umfasst.

14. System nach einem der Ansprüche 1 bis 12, wobei die Testsensorbank (1B, 10) eine Karte von zwei oder mehr Sensoren umfasst.

15. System nach einem der vorhergehenden Ansprüche, wobei die wiederbeschreibbare Speichervorrichtung eine erste wiederbeschreibbare Speichervorrichtung (18) ist und eine zweite wiederbeschreibbare Speichervorrichtung (48) vorgesehen ist.

16. System nach Anspruch 15, wobei die erste und/oder die zweite wiederbeschreibbare Speichervorrichtung (18, 48) auf einer Karte (30, 40) befestigt ist.

17. System nach Anspruch 16, wobei die Karte (30, 40) im Wesentlichen die Größe und die Form einer Kreditkarte besitzt.

18. System nach Anspruch 5, wobei das Substrat eine im Wesentlichen kreisförmige Form hat.

19. System nach einem der vorhergehenden Ansprüche, wobei die Testsensoren Teststreifen (17) sind.

20. System nach einem der vorhergehenden Ansprüche, wobei die Testsensoren siebgedruckt sind.

21. System nach einem der vorhergehenden Ansprüche, wobei das Messgerät Mittel zum Durchführen einer Untersuchung umfasst.

22. System nach einem der vorhergehenden Ansprüche, wobei die mindestens eine wiederbeschreibbare Speichervorrichtung (1C, 18) einen EEPROM oder einen Smartchip umfasst.

23. System nach einem der vorhergehenden Ansprüche, wobei mindestens ein wiederbeschreibbarer Speicher einen Mikroprozessor oder einen Mikrocontroller umfasst.

24. System nach einem der vorhergehenden Ansprüche, wobei mindestens eine wiederbeschreibbare Speichervorrichtung einen Näherungsspeicher umfasst.

25. System nach einem der Ansprüche 22 bis 24, wobei die Speichervorrichtung eine Speichergröße von nicht weniger als 1 K Bit, zum Beispiel ausgewählt aus der Gruppe von 1 K Bits, 2 K Bits, 4 K Bits, 8 K Bits, 16 K Bits, 32 K Bits, 64 K Bits, 128 K Bits, 256 K Bits, 512 K Bits, 1024 K Bits, besitzt.

26. System nach einem der vorhergehenden Ansprüche, wobei der Analyt Glukose ist.

27. System nach einem der vorhergehenden Ansprüche, wobei zumindest eine Information, die aus der Gruppe von
Kalibrierungscode,
Anzahl der nicht verwendeten oder verwendbaren Sensoren,
Sensorbank offen (wenn die Sensorbank geöffnet wurde)
Haltbarkeitsverfall,
Anweisungen für Benutzer,
Software, Korrekturen oder Upgrades, Ländercode,
Länder- oder Marktgeschmacksinformationen Personalisierungsinformationen,
selbstlernende Parameter,
erweiterte/aktualisierte
Analytberechnungsalgorithmusparameter,
vorherige Testergebnisse,
CHECKSUM (Überprüfen der Speicherinhalte), Parameter für die Benutzeroberfläche, Informationen über neue Produkte,
Informationen über Leistungen eines Produkts ausgewählt ist, von der wiederbeschreibbaren Speichervorrichtung in das Messgerät herunterladbar ist.

28. System nach einem der vorhergehenden Ansprüche, wobei zumindest eine Information, die aus der Gruppe von
Kalibrierungscode,
Anzahl der nicht verwendeten oder verwendbaren Sensoren,
Sensorbank offen (wenn die Sensorbank geöffnet wurde)
Haltbarkeitsverfall,
Anweisungen für Benutzer,
Software, Korrekturen oder Upgrades, Ländercode,
Länder- oder Marktgeschmacksinformationen Personalisierungsinformationen,
selbstlernende Parameter erweiterte/aktualisierte Analytberechnungsparameter,
vorherige Testergebnisse,
CHECKSUM (Überprüfen der Speicherinhalte), Parameter für die Benutzeroberfläche, Informationen über neue Produkte,
Informationen über Leistungen eines Produkts ausgewählt ist, in die wiederbeschreibbare Speichervorrichtung herunterladbar ist.

29. System nach einem der vorhergehenden Ansprüche, wobei zumindest eine Information, die aus der Gruppe von
Kalibrierungscode,
Anzahl der nicht verwendeten oder verwendbaren Sensoren,
Sensorbank offen (wenn die Sensorbank geöffnet wurde)
Haltbarkeitsverfall,
Anweisungen für Benutzer,
Software, Korrekturen oder Upgrades, Ländercode,
Länder- oder Marktgeschmacksinformationen Personalisierungsinformationen,
selbstlernende Parameter erweiterte/aktualisierte Analytberechnungsalgorithmusparameter,
vorherige Testergebnisse,
CHECKSUM (Überprüfen der Speicherinhalte), Parameter für die Benutzeroberfläche, Informationen über neue Produkte,
Informationen über Leistungen eines Produkts ausgewählt ist, in eine zweite wiederbeschreibbare Speichervorrichtung (48) heraufladbar oder herunterladbar ist.

30. Verfahren zum Durchführen einer Untersuchung auf ein Analyt in einem physiologischen Fluid, das umfasst, ein System (2B) nach einem der Ansprüche 1 bis 29 bereitzustellen; das System zu verwenden, um eine Untersuchung auf ein Analyt in einem physiologischen Fluid durchzuführen; und zumindest eine Information, die aus der Gruppe von
Kalibrierungscode,
Anzahl der nicht verwendeten oder verwendbaren Sensoren,
Sensorbank offen (wenn die Sensorbank geöffnet wurde)
Haltbarkeitsverfall,
Anweisungen für Benutzer,
Software, Korrekturen oder Upgrades, Ländercode,
Länder- oder Marktgeschmacksinformationen Personalisierungsinformationen,
selbstlernende Parameter erweiterte/aktualisierte Analytberechnungsalgorithmusparameter,
vorherige Testergebnisse,
CHECKSUM (Überprüfen der Speicherinhalte), Parameter für die Benutzeroberfläche, Informationen über neue Produkte,
Informationen über Leistungen eines Produkts ausgewählt ist, von zumindest der ersten wiederbeschreibbaren Speichervorrichtung (1C, 18) in das Messgerät (1A, 20) heraufzuladen.

31. Verfahren nach Anspruch 30, das umfasst, von dem Messgerät (1A, 20) zumindest eine Information, die aus der Gruppe von
Kalibrierungscode,
Anzahl der nicht verwendeten oder verwendbaren Sensoren,
Sensorbank offen (wenn die Sensorbank geöffnet wurde)
Haltbarkeitsverfall,
Anweisungen für Benutzer,
Software, Korrekturen oder Upgrades, Ländercode,
Länder- oder Marktgeschmacksinformationen Personalisierungsinformationen,
selbstlernende Parameter,
erweiterte/aktualisierte
Analytberechnungsalgorithmusparameter,
vorherige Testergebnisse,
CHECKSUM (Überprüfen der Speicherinhalte), Parameter für die Benutzeroberfläche, Informationen über neue Produkte,
Informationen über Leistungen eines Produkts ausgewählt ist, in zumindest die erste wiederbeschreibbare Speichervorrichtung (1C, 18) herunterzuladen.

32. Verfahren zum Durchführen einer Untersuchung auf ein Analyt in einem physiologischen Fluid, das umfasst, ein System nach einem der Ansprüche 1 bis 29 bereitzustellen; das System zu verwenden, um eine Untersuchung auf ein Analyt in einem physiologischen Fluid durchzuführen; und zumindest eine Information, die aus der Gruppe von
Kalibrierungscode,
Anzahl der nicht verwendeten oder verwendbaren Sensoren,
Sensorbank offen (wenn die Sensorbank geöffnet wurde)
Haltbarkeitsverfall,
Anweisungen für Benutzer,
Software, Korrekturen oder Upgrades, Ländercode,
Länder- oder Marktgeschmacksinformationen Personalisierungsinformationen,
selbstlernende Parameter,
erweiterte/aktualisierte
Analytberechnungsalgorithmusparameter,
vorherige Testergebnisse,
CHECKSUM (Überprüfen der Speicherinhalte), Parameter für die Benutzeroberfläche, Informationen über neue Produkte,
Informationen über Leistungen eines Produkts ausgewählt ist, von zumindest der zweiten wiederbeschreibbaren Speichervorrichtung (48) in das Messgerät (1A, 20) heraufzuladen.

33. Verfahren nach Anspruch 32, das umfasst, von dem Messgerät (1A, 20) zumindest eine Information, die aus der Gruppe von
Kalibrierungscode,
Anzahl der nicht verwendeten oder verwendbaren Sensoren,
Sensorbank offen (wenn die Sensorbank geöffnet wurde)
Haltbarkeitsverfall,
Anweisungen für Benutzer,
Software, Korrekturen oder Upgrades, Ländercode,
Länder- oder Marktgeschmacksinformationen Personalisierungsinformationen,
selbstlernende Parameter erweiterte/aktualisierte Analytberechnungsalgorithmusparameter,
vorherige Testergebnisse,
CHECKSUM (Überprüfen der Speicherinhalte), Parameter für die Benutzeroberfläche, Informationen über neue Produkte,
Informationen über Leistungen eines Produkts ausgewählt ist, in zumindest die zweite wiederbeschreibbare Speichervorrichtung (48) herunterzuladen.

34. Verfahren nach einem der Ansprüche 30 bis 33, wobei ein für eine Untersuchung verwendeter Testsensor während einer Untersuchung innerhalb der Sensorbank (1B, 10) verbleibt.

35. Verfahren nach einem der Ansprüche 30 bis 34, wobei ein Testsensor während einer Untersuchung zumindest teilweise von der Sensorbank für die Verwendung entfernt wird.

36. Verfahren nach Anspruch 35, das umfasst, einen Sensor für eine Untersuchung an einen Testanschluss zu liefern.

37. Verfahren nach einem der Ansprüche 30 bis 36, das umfasst, die Konzentration eines Analyts zu messen.

## Revendications

1. Système (2B) destiné à conduire un test de présence d'un analyte dans un fluide physiologique, le système présentant :
un banc (1B, 10) de capteurs de test qui comprend au moins initialement plus d'un capteur de test (17), pour tester un analyte,
un dispositif (1C, 18) à mémoire ré-inscriptible associé audit banc de capteurs de test,
un dispositif de mesure (1A, 20) qui conduit un test en recourant à un capteur de test du banc,
le dispositif de mesure comprenant des moyens de lecture qui lisent des informations dans le dispositif à mémoire réinscriptible et un moyen d'écriture qui écrit des informations dans le dispositif à mémoire ré-inscriptible et
des moyens de transmission (100B, 102B) qui transmettent des informations entre la mémoire ré-inscriptible et le dispositif de mesure,
**caractérisé en ce que**
le banc de capteurs de test et le dispositif à mémoire ré-inscriptible comprennent chacun un code d'identification ("XXXX") unique, le dispositif de mesure étant configuré pour échanger des informations avec le dispositif à mémoire ré-inscriptible uniquement si le dispositif de mesure détecte que le dispositif à mémoire ré-inscriptible et le banc de capteurs de test ont un code d'identification unique commun.

2. Système selon la revendication 1, dans lequel le dispositif (1C, 18) à mémoire ré-inscriptible est associé de manière unique et/ou permanente au banc (1B, 10) de capteurs de test.

3. Système selon les revendications 1 ou 2, dans lequel le dispositif (1C, 18) à mémoire ré-inscriptible est relié physiquement au banc (1B, 10) de capteurs de test.

4. Système selon la revendication 3, dans lequel le dispositif (1C, 18) à mémoire ré-inscriptible est attaché fixement au banc (1B, 10) de capteurs de test.

5. Système selon la revendication 4, dans lequel le banc de capteurs de test comprend un logement ou un support et le dispositif (1C, 18) à mémoire ré-inscriptible est collé au boîtier ou au support en utilisant un adhésif.

6. Système selon les revendications 4 ou 5, dans lequel le dispositif (1C, 18) à mémoire ré-inscriptible est collé en utilisant un adhésif durcissant à froid, un adhésif à chaud, un adhésif sensible à la pression ou un adhésif fusible à chaud.

7. Système selon les revendications 5 ou 6, dans lequel le logement comprend une matière plastique telle que le polypropylène ou une matière plastique à base de polypropylène.

8. Système selon les revendications 5 ou 6, dans lequel le support comprend une matière plastique telle que le polyester ou une matière plastique à base de polypropylène.

9. Système selon la revendication 1, dans lequel le dispositif (1C, 18) à mémoire ré-inscriptible est raccordé sans fil au banc (1B, 10) de capteurs de test en utilisant une transmission RF (à fréquence radio).

10. Système selon la revendication 9, présentant sur le banc (1B, 10) de capteurs de test et sur la mémoire ré-inscriptible (1C, 18) des moyens qui permettent de transmettre le code d'identification unique ("XXXX") au dispositif de mesure (1A, 20).

11. Système selon l'une quelconque des revendications précédentes, dans lequel le banc (1B, 10) de capteurs de test peut être raccordé physiquement de manière libérable au dispositif de mesure (1A, 20).

12. Système selon la revendication 11, dans lequel le banc (1B, 10) de capteurs de test peut être inséré au moins en partie dans le dispositif de mesure (1A, 20).

13. Système selon l'une quelconque des revendications précédentes, dans lequel le banc (1B, 10) de capteurs de test comprend un empilement de deux ou plusieurs capteurs.

14. Système selon l'une quelconque des revendications 1 à 12, dans lequel le banc (1B, 10) de capteurs de test comprend une carte constituée de deux ou plusieurs capteurs.

15. Système selon l'une quelconque des revendications précédentes, dans lequel le dispositif à mémoire ré-inscriptible est un premier dispositif (18) à mémoire ré-inscriptible et dans lequel un deuxième dispositif (48) à mémoire ré-inscriptible est prévu.

16. Système selon la revendication 15, dans lequel le premier et/ou le deuxième dispositif (18, 48) à mémoire ré-inscriptible sont montés sur une carte (30, 40).

17. Système selon la revendication 16, dans lequel la carte (30, 40) présente essentiellement la taille et la forme d'une carte de crédit.

18. Système selon la revendication 5, dans lequel le support a une forme essentiellement circulaire.

19. Système selon l'une quelconque des revendications précédentes, dans lequel les capteurs de test sont des rubans de test (17).

20. Système selon l'une quelconque des revendications précédentes, dans lequel les capteurs de test sont sérigraphiés.

21. Système selon l'une quelconque des revendications précédentes, dans lequel le dispositif de mesure comprend des moyens permettant de conduire un test.

22. Système selon l'une quelconque des revendications précédentes, dans lequel au moins un dispositif (1C, 18) à mémoire ré-inscriptible comprend une EEPROM ou une puce intelligente.

23. Système selon l'une quelconque des revendications précédentes, dans lequel au moins une mémoire ré-inscriptible comprend un microprocesseur ou un microcontrôleur.

24. Système selon l'une quelconque des revendications précédentes, dans lequel au moins un dispositif à mémoire ré-inscriptible comprend une mémoire de proximité.

25. Système selon l'une quelconque des revendications 22 à 24, dans lequel le dispositif à mémoire présente une mémoire d'une taille non inférieure à 1K bit, et par exemple une taille sélectionnée dans l'ensemble des tailles de 1K bits, 2K bits, 4K bits, 8K bits, 16K bits, 32K bits, 64K bits, 128K bits, 256K bits, 512K bits et 1024K bits.

26. Système selon l'une quelconque des revendications précédentes, dans lequel l'analyte est le glucose.

27. Système selon l'une quelconque des revendications précédentes, dans lequel au moins un élément d'information sélectionné dans l'ensemble constitué de :
un code d'étalonnage
le nombre de capteurs inutilisés ou utilisables
banc de capteurs ouvert (lorsque le banc de capteur a été ouvert)
durée de conservation expirée
instructions pour l'utilisateur
logiciel, corrections ou mises à jour code de pays
information de pays ou d'arôme de marché informations de personnalisation
paramètres d'auto-apprentissage
paramètres mise à jour d'algorithmes de calcul d'analyte
résultats de tests précédents
CHECKSUM (vérification du contenu de la mémoire) paramètres de l'interface d'utilisateur informations concernant de nouveaux produits
informations concernant les performances du produit peut être téléchargé depuis ledit dispositif à mémoire ré-inscriptible jusque sur le dispositif de mesure.

28. Système selon l'une quelconque des revendications précédentes, dans lequel au moins un élément d'information sélectionné dans l'ensemble constitué de :
un code d'étalonnage
le nombre de capteurs inutilisés ou utilisables
banc de capteurs ouvert (lorsque le banc de capteur a été ouvert)
durée de conservation expirée
instructions pour l'utilisateur
logiciel, corrections ou mises à jour code de pays
information de pays ou d'arôme de marché informations de personnalisation
paramètres d'auto-apprentissage
paramètres mise à jour d'algorithmes de calcul d'analyte
résultats de tests précédents
CHECKSUM (vérification du contenu de la mémoire) paramètres de l'interface d'utilisateur informations concernant de nouveaux produits informations concernant les performances du produit
peut être téléchargé sur ledit dispositif à mémoire ré-inscriptible.

29. Système selon l'une quelconque des revendications précédentes, dans lequel au moins un élément d'information sélectionné dans l'ensemble constitué de :
un code d'étalonnage
le nombre de capteurs inutilisés ou utilisables banc de capteurs ouvert (lorsque le banc de capteur a été ouvert)
durée de conservation expirée
instructions pour l'utilisateur
logiciel, corrections ou mises à jour code de pays
information de pays ou d'arôme de marché informations de personnalisation
paramètres d'auto-apprentissage
paramètres mise à jour d'algorithmes de calcul d'analyte
résultats de tests précédents
CHECKSUM (vérification du contenu de la mémoire) paramètres de l'interface d'utilisateur informations concernant de nouveaux produits informations concernant les performances du produit
peut être téléchargé depuis ou vers un deuxième dispositif (48) à mémoire ré-inscriptible.

30. Procédé pour conduire un test d'un analyte présent dans un fluide physiologique, et comprenant l'étape qui consiste à prévoir un système (2B) selon l'une quelconque des revendications 1 à 29, à utiliser le système pour conduire un test sur un analyte présent dans un fluide physiologique et à télécharger au moins un élément d'information sélectionné dans l'ensemble constitué de :
un code d'étalonnage
le nombre de capteurs inutilisés ou utilisables
banc de capteurs ouvert (lorsque le banc de capteur a été ouvert)
durée de conservation expirée
instructions pour l'utilisateur
logiciel, corrections ou mises à jour code de pays
information de pays ou d'arôme de marché informations de personnalisation
paramètres d'auto-apprentissage
paramètres mise à jour d'algorithmes de calcul d'analyte
résultats de tests précédents
CHECKSUM (vérification du contenu de la mémoire) paramètres de l'interface d'utilisateur informations concernant de nouveaux produits informations concernant les performances du produit
depuis au moins ledit premier dispositif (1C, 18) à mémoire ré-inscriptible jusque sur le dispositif de mesure (1A, 20).

31. Procédé selon la revendication 30, comprenant l'étape qui consiste à télécharger depuis le dispositif de mesure (1A, 20) au moins un élément d'information sélectionné dans l'ensemble constitué de :
un code d'étalonnage
le nombre de capteurs inutilisés ou utilisables
banc de capteurs ouvert (lorsque le banc de capteur a été ouvert)
durée de conservation expirée
instructions pour l'utilisateur
logiciel, corrections ou mises à jour code de pays
information de pays ou d'arôme de marché informations de personnalisation
paramètres d'auto-apprentissage
paramètres mise à jour d'algorithmes de calcul d'analyte
résultats de tests précédents
CHECKSUM (vérification du contenu de la mémoire) paramètres de l'interface d'utilisateur informations concernant de nouveaux produits informations concernant les performances du produit sur au moins ledit premier dispositif (1C, 18) à mémoire ré-inscriptible.

32. Procédé pour conduire un test d'un analyte présent dans un fluide physiologique, et comprenant l'étape qui consiste à prévoir un système selon l'une quelconque des revendications 1 à 29, à utiliser le système pour conduire un test sur un analyte présent dans un fluide physiologique et à télécharger au moins un élément d'information sélectionné dans l'ensemble constitué de :
un code d'étalonnage
le nombre de capteurs inutilisés ou utilisables
banc de capteurs ouvert (lorsque le banc de capteur a été ouvert)
durée de conservation expirée
instructions pour l'utilisateur
logiciel, corrections ou mises à jour code de pays
information de pays ou d'arôme de marché informations de personnalisation
paramètres d'auto-apprentissage
paramètres mise à jour d'algorithmes de calcul d'analyte
résultats de tests précédents
CHECKSUM (vérification du contenu de la mémoire) paramètres de l'interface d'utilisateur informations concernant de nouveaux produits informations concernant les performances du produit
depuis au moins ledit premier dispositif (1C, 18) à mémoire ré-inscriptible jusque sur le dispositif de mesure (1A, 20).depuis au moins ledit deuxième dispositif (48) à mémoire ré-inscriptible jusque sur le dispositif de mesure (1A, 20).

33. Procédé selon la revendication 32, comprenant l'étape qui consiste à télécharger depuis le dispositif de mesure (1A, 20) au moins un élément d'information sélectionné dans l'ensemble constitué de :
un code d'étalonnage
le nombre de capteurs inutilisés ou utilisables
banc de capteurs ouvert (lorsque le banc de capteur a été ouvert)
durée de conservation expirée
instructions pour l'utilisateur
logiciel, corrections ou mises à jour code de pays
information de pays ou d'arôme de marché informations de personnalisation
paramètres d'auto-apprentissage
paramètres mise à jour d'algorithmes de calcul d'analyte
résultats de tests précédents
CHECKSUM (vérification du contenu de la mémoire) paramètres de l'interface d'utilisateur informations concernant de nouveaux produits informations concernant les performances du produit
jusque sur au moins ledit deuxième dispositif (48) mémoire ré-inscriptible.

34. Procédé selon l'une quelconque des revendications 30 à 33, dans lequel un capteur de test utilisé pour un test reste à l'intérieur du banc (1B, 10) de capteurs pendant un test.

35. Procédé selon l'une quelconque des revendications 30 à 34, dans lequel un capteur de test est enlevé au moins en partie du banc de capteurs pour être utilisé pendant un test.

36. Procédé selon la revendication 35, comprenant la fourniture d'un capteur sur un orifice de test en vue d'effectuer un test.

37. Procédé selon l'une quelconque des revendications 30 à 36, comprenant la mesure de la concentration d'un analyte.
